# EUROPEAN PATENT SPECIFICATION

(11) **EP 0 416 038 B1**
(45) Date of publication and mention of the grant of the patent: **26.03.1997**
(21) Application number: 89907947.9
(22) Date of filing: 28.06.1989
(51) Int. Cl.: C07C 39/14, C07C 43/225, C07C 43/23, C07C 49/788, C07D 321/00, C07F 7/18, C07F 9/12, C12Q 1/00, C12Q 1/70, G01N 21/76, G01N 33/542, G01N 33/554, G01N 33/00

(54) **NOVEL CHEMILUMINESCENT FUSED POLYCYCLIC RING-CONTAINING 1,2-DIOXETANES AND ASSAYS IN WHICH THEY ARE USED**
CHEMILUMINESZIERENDE 1,2- DIOXETANE MIT KONDENSIERTEN POLYZYKLISCHEN RINGEN.
NOUVEAUX 1,2-DIOXETANES CHIMIOLUMINESCENTS CONTENANT UN ANNEAU POLYCYCLIQUE FUSIONNE, ET ANALYSES LES UTILISANT

(30) Priority: 30.06.1988 US 213672
(43) Date of publication of application: 13.03.1991
(73) Proprietor: Tropix, Inc. (a Delaware corporation), Bedford, MA 01730 (US)
(72) Inventor: EDWARDS, Brooks, Cambridge, MA 02138-1377 (US); BRONSTEIN, Irena, Y., Newton, MA 02158 (US); LAIRD, Alison, A., North Andover, MA 01845-4727 (US); VOYTA, John, C., North Reading, MA 01864 (US)
(74) Representative: Goldin, Douglas Michael
(86) International application number: PCT/US89/02793
(87) International publication number: WO 90/00164

(56) References cited:
- EP-A- 0 254 051
- EP-A- 0 254 051
- WO-A-89/06226
- JP-B- 7 306 526
- JP-B- 7 306 526
- TETRAHEDRON LETTERS, vol. 28, no. 44, 1987, pages 5319-5322, Pergamon Journals
- Ltd, Oxford, GB; R. CURCI et al.: "Synthesis of 1,2-dioxetanes via electron- transfer oxygenation"
- Tetrahedron Letters, Vol. 28 No.11, Schaap et al (1987), pages 1155-1158 - see page 1155.
- Chemical Abstracts Vol. 81, No. 9, 02-09-1974, Hellwinkel et al, abstract nr. 49097y
- Chemical Abstracts, Vol. 87 Nr. 13, 26-09-1977, Abstract Nr. 102512g

## Description

### FIELD OF THE INVENTION

This invention relates to improved chemiluminescent 1,2-dioxetane compounds and novel intermediates used in their preparation. More particularly, this invention relates to improved chemiluminescent 1,2-dioxetane compounds that contain labile groups removable enzymatically, chemically or electrochemically to produce fluorophores that in turn emit optically detectable energy, usually luminescence in the form of visible light, and that also contain stabilizing groups that prevent these dioxetanes from decomposing before the labile group comes in contact with the substance(s) that will remove it. This invention further relates to the incorporation of such light-emitting reactants in art-recognized immunoassays, chemical assays and nucleic acid probe assays, and to their use as direct chemical/physical probes, to permit an analyte, a chemical or biological substance whose presence, amount or structure is being determined, to be identified or quantified.

### BACKGROUND OF THE INVENTION

Chemiluminescent 1,2-dioxetanes and their use in previously-developed chemical and biochemical assays has assumed increasing importance in recent years, particularly with the advent of enzymatically-cleavable 1,2-dioxetanes; see, for example, copending Bronstein U.S. patent application Serial No. 889,823, "Method of Detecting a Substance Using Enzymatically-Induced Decomposition of Dioxetanes", filed July 24, 1986; Bronstein et al U.S. patent application Serial No. 140,035, "Dioxetanes for Use in Assays", filed December 31, 1987; Edwards U.S. patent application Serial No. 140,197, "Synthesis of 1,2-Dioxetanes and Intermediates Therefor", filed December 31, 1987 and Voyta et al U.S. patent application Serial No. 203,263, "Chemiluminescence Enhancement", filed June 1, 1988.

It is known that chemiluminescent 1,2-dioxetanes substituted at the 4-position on the dioxetane ring with a monocyclic aromatic ring-containing fluorophore moiety can be configured to maximize their charge transfer characteristics ("emission efficiencies") in the excited state and, as a result, increase the intensity of the light emitted when such compounds decompose. This is the so-called "meta effect" discussed in Schaap et al, Tetrahedron Letters, Vol 28, No 11, p. 1155 - 1158 (1987). The meta effect, as the name implies, is achieved by placing a labile substituent containing a bond cleavable to yield an electron-rich fluorophore moiety meta to the point of attachment of the monocyclic aromatic ring to the dioxetane ring.

Knowledge of the meta effect, however, would not have enabled those skilled in the art to predict with assurance what effect, if any, substituent placement might have on the intensity of light emitted by the decomposition of chemiluminescent 1,2-dioxetanes substituted at the 4-position on the dioxetane ring with fused polycyclic, rather than monocyclic, ring-containing fluorophore moieties, and certainly would give no guidance whatsoever regarding the duration or the nature, i.e., the wavelength or color, of light emitted by such polycyclic moieties on decomposition.

EP-A-254051 describes chemiluminescent 1,2-dioxetane compounds and their use. None of the compounds described is substituted as required according to the present claims.

Tetrahedron Letters, vol 28, no. 44, pages 5319-5322 (1987) similarly describes certain 1,2-dioxetane compounds.

WO-A-89/06226, published on 13 July 1989 and claiming priority from Edwards US Patent Application Serial No. 140197 relates to 1,2-dioxetanes and their use.

### SUMMARY OF THE INVENTION

This invention is concerned with chemiluminescent 1,2-dioxetanes substituted with certain fused polycyclic ring-containing fluorophore moieties. In particular, it is concerned with chemiluminescent 1,2-dioxetanes stabilized at the 3-position on the dioxetane ring against decomposition prior to the molecule's coming in contact with a labile group-removing substance (e.g., an enzyme that will result in enzymatic cleavage of the dioxetane molecule) and substituted at the 4-position on the dioxetane ring with a naphthalene residue which is itself substituted in a particular fashion with a labile ring substituent containing a bond which, when cleaved, renders this naphthalene residue electron-rich and hence renders the dioxetane decomposable to emit light.

It has now been discovered, quite unexpectedly, that if such fused polycyclic ring-containing fluorophore moieties are substituted in a particular pattern, i.e., if they contain at a particular ring position in relation to the naphthalene residue's point(s) of attachment to the dioxetane ring a labile ring substituent having a cleavable bond they will, by virtue of this substitution pattern, decompose to emit light of unexpectedly greater intensity in certain environments, as well as light of longer duration, than that emitted by corresponding isomeric compounds, i.e., otherwise identical fused polycyclic ring-substituted 1,2-dioxetanes having the same labile ring substituents on the polycyclic ring but on different ring positions and thus in a different substitution pattern.

In contrast to other chemiluminescent materials -- for example acridinium esters, or luminols under most conditions -- the chemiluminescent 1,2-dioxetanes of this invention, when decomposed, emit light as a glow rather than a flash. The kinetic difference between these two types of light emission is illustrated in the following excerpt from Knox Van Dyke, "Bioluminescence and Chemiluminescence: Instruments and Applications" (Boca Raton, Fla.: CRC Press, 1985), Vol. 1, p. 5:

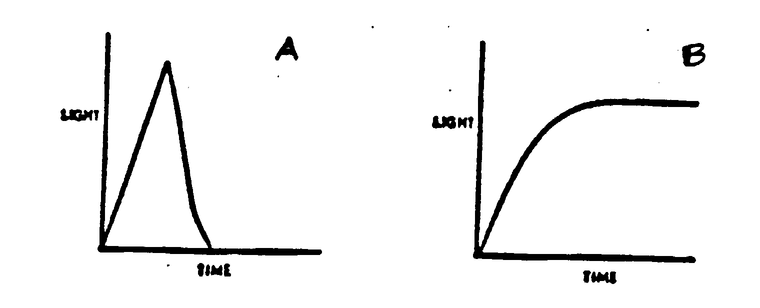

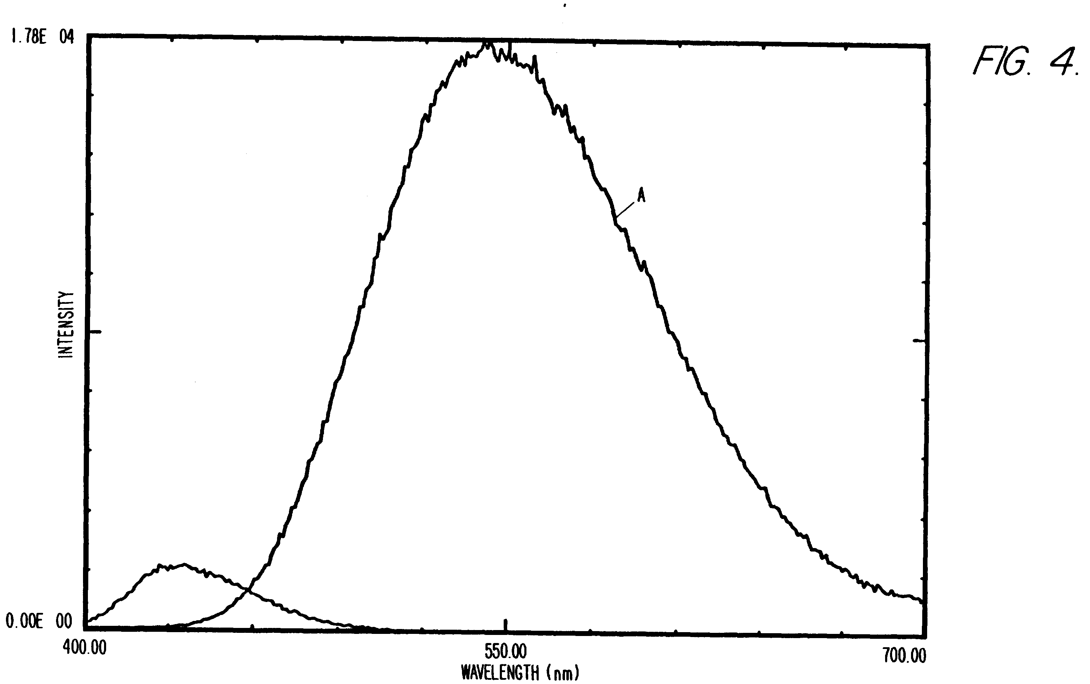

FIGURE 4. The production of light from chemical reaction that produces a light peak or flash is depicted in (A) while a steady-state production of light is depicted in (B). In general, the measurement of light in state (B) is more convenient and reproducible than in state (A).

High intensity light emission permits the use of simple photodetectors (silicon photodiodes or photographic film, for example) as well as conventionally employed photomultiplier tubes to detect the emitted light. The stability of the emission signal produced by these enhanced intensity chemiluminescent 1,2-dioxetanes also simplifies assay procedures in which they are used as the means by which the analyte is identified or quantified because the chemiluminescent reaction can be initiated prior to transferring the assay mixture to the light detector. Hence, precisely timed operations are not required.

This invention's substitution pattern is one in which the labile ring substituent's point of attachment to the naphthalene residue, in relation to this residue's point of attachment to the dioxetane ring, is such that the total number of ring atoms, e.g., ring carbon atoms, separating these two points of attachment, including the carbon atoms at the points of attachment, is an odd whole number. Preferably, the total number of ring atoms separating these two points of attachment, including the ring atoms at the points of attachment, will be an odd whole number of 5 or greater. A naphthalene residue attached by a single bond to the dioxetane ring at this residue's 2-carbon atom and bearing an enzyme-cleavable substituent, e.g., a phosphate ester group, at its 5- or 7-carbon atom, or a naphthalene residue attached at its 1-carbon atom and substituted at its 8-carbon atom, are typical examples from among this invention's contemplated odd pattern fused polycyclic aromatic hydrocarbon ring-containing fluorophore moieties.

Most unexpectedly, the novel odd pattern substituted fused polycyclic ring-containing 1,2-dioxetanes of this invention also exhibit on decomposition a pronounced shift in the wavelength of the light emitted, i.e., this light is a different color from that emitted by corresponding isomeric compounds which have an even rather than an odd numbered substitution pattern.

This invention is further concerned with the use of these improved chemiluminescent 1,2-dioxetanes in art-recognized immunoassays, chemical assays and nucleic acid probe assays to detect the presence or determine the concentration of chemical or biological substances, and as direct chemical/physical probes for studying the molecular structures or microstructures of various macromolecules: synthetic polymers, proteins, nucleic acids and the like.

Also, by using either two or more of the improved chemiluminescent 1,2-dioxetanes of this invention, each of which upon decomposition emits light of a different wavelength, or one or more of these compounds together with a different chemiluminescent compound, such as one of the enzymatically decomposable 1,2-dioxetanes disclosed in the aforementioned copending Bronstein, Bronstein et al and Edwards applications, or a chemically or electrochemically cleavable analog thereof, which emits light of yet another wavelength, each of such compounds being structured so as to be decomposable by a different means (e.g., one of a pair of such compounds can contain a phosphate ester group cleavable by a phosphatase and the other an α-D- or β-D-glucoside group cleavable by β-glucosidase, or one compound can contain an enzyme-cleavable group and the other a chemically cleavable group such as a hydroxyl group, an alkanoyl or aroyl ester group, or an alkyl or aryl silyloxy group), a multi-channel assay can be designed in which different cleaving means attached to or associated with two or more different analytes will, by cleaving different cleavable dioxetane substituents, induce the emission of light of a different wavelength for each analyte being assayed.

It is, therefore, an object of this invention to provide improved chemiluminescent 1,2-dioxetane compounds and novel intermediates used in preparing them.

A further object of this invention is to provide chemiluminescent 1,2-dioxetanes stabilized at the 3-position on the dioxetane ring against decomposition prior to the molecule's being brought into contact with a labile group-removing substance to intentionally cleave a labile substituent's bond, and substituted at the 4-position on the dioxetane ring with a fused polycyclic ring-containing fluorophore moiety which is itself substituted with a labile ring substituent containing a bond which, when cleaved, renders this fluorophore moiety electronrich and hence renders the dioxetane decomposable to emit light, this labile ring substituent's point of attachment to the fused polycyclic ring, in relation to this ring's point of attachment to the dioxetane ring, being such that the total number of ring atoms separating these two points of attachment, including the ring atoms at the points of attachment, is an odd whole number.

Another object of this invention is to provide improved chemiluminescent 1,2-dioxetane compounds which, upon decomposition, emit light of greater intensity than the light emitted by isomers thereof and which also exhibit a shift in the wavelength of the light emitted as compared to the wavelengths of light emitted by the corresponding even pattern substituted compounds.

A still further object of this invention is the use of these improved chemiluminescent 1,2-dioxetane compounds to generate light in aqueous and non-aqueous media.

Yet another object of this invention is the use of these improved chemiluminescent 1,2-dioxetane compounds in art-recognized immunoassays, chemical assays and nucleic acid probe assays, including multi-channel assays, to detect the presence or determine the concentration of chemical or biological substances, and as direct chemical/physical probes for studying the molecular structures or microstructures of various macromolecules: synthetic polymers, proteins, nucleic acids and the like.

These and other objects, as well as the nature, scope and utilization of this invention, will become readily apparent to those skilled in the art from the following description, the drawings and the appended claims.

### BRIEF DESCRIPTION OF THE DRAWINGS

FIG. 1 shows the hyperchromic effect and bathochromically shifted emission by 3-(2'-spiroadamantane)-4-methoxy-4-(7"-acetoxy)naphth-2'-yl-1,2-dioxetane, prepared as described in Example I infra, when decomposed with carboxylesterase as described in Example II, infra.

FIG. 2 compares the intensity and wavelength of the light emitted by 3-(2'-spiroadamantane)-4-methoxy-4-(7"-acetoxy)naphth-2'-yl-1,2-dioxetane("A") with that of its 2,6-naphthalene dioxetane acetate isomer when each of these isomers is decomposed with aqueous sodium hydroxide as described in Example III, infra.

FIG. 3 compares the intensity and wavelength of the light emitted by 3-(2'spiroadamantane)-4-methoxy-4-(7"-acetoxy)naphth-2'-yl-1,2-dioxetane ("A") with that of 3-(2'-spiroadamantane)-4-methoxy-4-(3"-acetoxy)phenyl -1,2-dioxetane when each is decomposed with aqueous sodium hydroxide solution as also described in Example III, infra.

FIG. 4 compares the intensity and wavelength of the light emitted by the 2,7-naphthalene dioxetane acetate of Example I ("A") with that of its 2,6-isomer when each of these isomers is decomposed with sodium hydroxide in aqueous methyl cyanide as described in Example IV, infra.

FIG. 5 compares the intensity and wavelength of the light emitted by the 2,7-naphthalene dioxetane acetate of Example I with that of its 2,6-isomer when each of these compounds is decomposed with sodium hydroxide in aqueous acetonitrile as described in Example V infra. In FIG. 5 "A" is the plot for the 2,7-isomer.

FIG. 6 compares the intensity and wavelength of the light emitted by the 2,7-naphthalene dioxetane acetate of Example I ("A") with that of 3-(2'-spiroadamantane)-4-methoxy-4-(3"-acetoxy)phenyl-1,2-dioxetane when each is decomposed in sodium methoxide as described in Example VI, infra.

FIGS. 7 and 8 show the duration of the light emitted when 3-(2'-spiroadamantane)-4-methoxy-4-(6"-acetoxy)naphth-2'-yl-1,2-dioxetane (FIG. 7) and its 2,7-isomer of this invention (FIG. 8) are decomposed with sodium hydroxide in aqueous CH₃CN as described in Example VII, infra.

### DETAILED DESCRIPTION OF THE INVENTION

The improved chemiluminescent 1,2-dioxetanes of 5 this invention can be represented by the general formula:

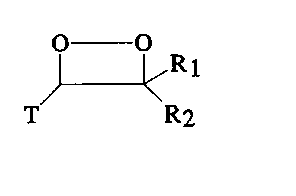

In this formula the symbol R₁ represents a C₁-C₂₀ alkoxy group. Preferably, R₁ is a methoxy group.

The symbol R₂ represents a naphthalene residue having a labile ring substituent containing a bond which, when cleaved, renders the naphthalene residue electron-rich to in turn render the dioxetane compound decomposable to emit light. This labile ring substituent's point of attachment to the naphthalene residue, in relation to this ring's point(s) of attachment to the dioxetane ring (single bond attachment), is such that the total number of ring atoms separating these points of attachment, including the ring atoms at the points of attachment, is an odd whole number.

Included among the naphthalene compounds whose residues can be used to form this fluorophore moiety are such as: 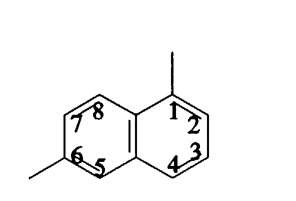 the substituent bonds indicating a 1,6-substitution pattern as in 3-(2'-spiroadamantane)-4-methoxy-4-(6"-disodiumphosphoryloxy)-naphth-1'-yl-1,2-dioxetane, as well as derivatives thereof substituted with one or more non-labile substituents such as a branched or straight chain alkyl group having 1 to 20 carbon atoms, inclusive, e.g., methyl, n-butyl or decyl, a branched or straight chain heteroalkyl group having 1 to 7 carbon atoms, inclusive, e.g., methoxy, hydroxyethyl or hydroxypropyl; an aryl group having 1 or 2 rings, e.g., phenyl; a heteroaryl group having 1 or 2 rings, e.g., pyrrolyl or pyrazolyl; a cycloalkyl group having 3 to 7 carbon atoms, inclusive, in the ring, e.g., cyclohexyl; a heterocycloalkyl group having 3 to 6 carbon atoms, inclusive, in the ring, e.g., dioxane; an aralkyl group having 1 or 2 rings, e.g., benzyl; an alkaryl group having 1 or 2 rings, e.g., tolyl; an electron-withdrawing group, such as a perfluoroalkyl group having between 1 and 7 carbon atoms, inclusive, e.g., trifluoromethyl; a halogen; CO₂H, ZCO₂H, SO₃H, NO₂, ZNO₂, C≡N, or ZC≡N, where Z is a branched or straight chain alkyl group having 1 to 7 carbon atoms, inclusive, e.g., methyl, or an aryl group having 1 or 2 rings, e.g., phenyl; an electron-donating group, e.g., a branched or straight chain C₁-C₇ alkoxy group, e.g., methoxy or ethoxy: an aralkoxy group having 1 or 2 rings, e.g., phenoxy; a branched or straight chain C₁-C₇ alkoxy group, e.g., methoxy or ethoxy; an aralkoxy group having 1 or 2 rings, e.g., phenoxy; a branched or straight chain C₁-C₇ hydroxyalkyl group, e.g., hydroxymethyl or hydroxyethyl; a hydroxyaryl group having 1 or 2 rings, e.g., hydroxyphenyl; a branched or straight chain C₁-C₇ alkyl ester group, e.g., acetate; an aryl ester group having 1 or 2 rings, e.g., benzoate; or a heteroaryl group having 1 or 2 rings, e.g., benzoxazole, benzthiazole, benzimidazole or benztriazole.

Included among the labile ring substituents which can be positioned on the naphthalene residue to make up the fluorophore moieties of this invention are substituents which, when cleaved, yield an oxygen anion, a sulfur anion, or a nitrogen anion such as a sulfonamido anion. Such substituents can be chemically cleavable: a hydroxyl group, an alkanoyl or aroyl ester group, or an alkyl or aryl silyloxy group, but preferably are enzymatically cleavable. Enzymatically cleavable substituents include phosphate ester groups represented by the general formula: 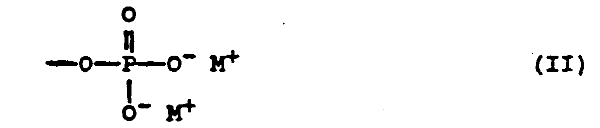 wherein M+ represents a cation such as alkali metal, e.g., sodium or potassium, ammonium, or a C₁-C₇ alkyl, aralkyl or aromatic quaternary ammonium cation, N(R₃)₄+ in which each R₃ can be alkyl, e.g., methyl or ethyl, aralkyl, e.g., benzyl, or form part of a heterocyclic ring system, e.g., pyridinium, and particularly the disodium salt. Such quaternary ammonium cations can also be connected through one of their quaternizing groups to a polymeric backbone, viz. 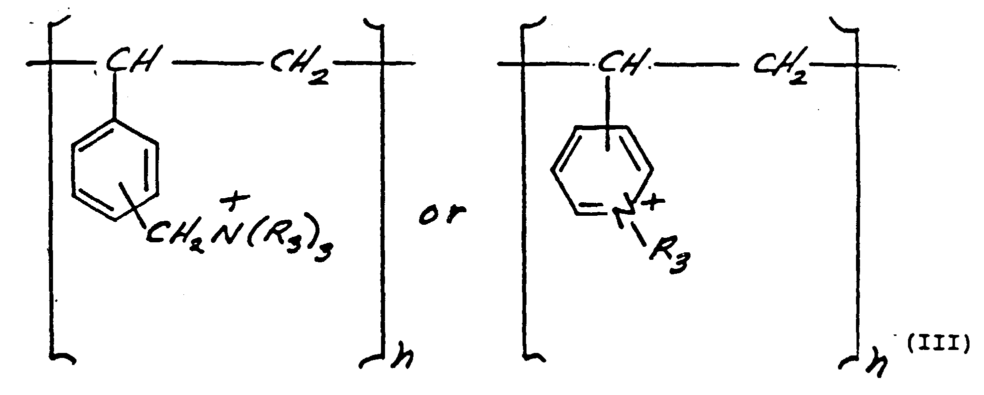 where n is greater than 1, or can be part of a polyquaternary ammonium salt, i.e., an ionene polymer.

Enzymatically cleavable substituents also include enzyme-cleavable alkanoyloxy groups, e.g., an acetate ester group, or an enzyme-cleavable oxacarboxylate group, 1-phospho-2,3-diacylglyceride group, 1-thio-D-glucoside group, adenosine triphosphate analog group, adenosine diphosphate analog group, adenosine monophosphate analog group, adenosine analog group, α-D-galactoside group, β-D-galactoside group, α-D-glucoside group, β-D-glucoside group, α-D-zannoside group, β-d-mannoside group, β-D-fructofuranoside group, β-D-glucosiduronate group, p-toluenesulfonyl-L-arginine ester group or p-toluenesulfonyl-L-arginine amide group.

An illustrative but by no means exhaustive listing of labile group-substituted naphthalene residue-containing fluorophore moieties represented by R₂ includes the following, numbered here as though they were substituents at the 4-position on the dioxetane ring: (5"-disodiumphosphoryloxy)naphth-2'-yl, (7"-disodiumphosphoryloxy)naphth-2'-yl, (6"-disodiumphosphoryloxy)naphth-1'-yl, (8"-disodiumphosphoryloxy)naphth-1'-yl, (5"-acetoxy) naphth-2'-yl, (7"-acetoxy)naphth-2'-yl, (6"-acetoxy)naphth-1'-yl, (8"-acetoxy)naphth-1'-yl, (5"-β-galactosyloxy)naphth-2'-yl, (7"-β-galactosyloxy)naphth-2'-yl, (6"-β-galactosyloxy)naphth-1'-yl, and the like.

The symbol T represents an adamantyl group. This stabilizing group prevents the dioxetane compound from decomposing before the bond in the labile ring substituent on the naphthalene residue-containing fluorophore moiety is intentionally cleaved. T may be e.g., an adamant-2-ylidene group.

One or more of the substituents R₁, R₂ and T can also include a substituent which enhances the water solubility of the 1,2-dioxetane, such as a carboxylic acid, e.g., acetic acid, sulfonic acid, e.g., methanesulfonic acid or ethanesulfonic acid, or quaternary amino salt group, e.g., ammonium bromide.

A preferred class of odd pattern substituted naphthalene residue-containing 1,2-dioxetanes coming within the scope of this invention are 3-(2'-spiroadamantane)-4-methoxy-4-(phosphoryloxy)naphth-2'-yl-1,2-dioxetane salts such as those represented by the formulas: 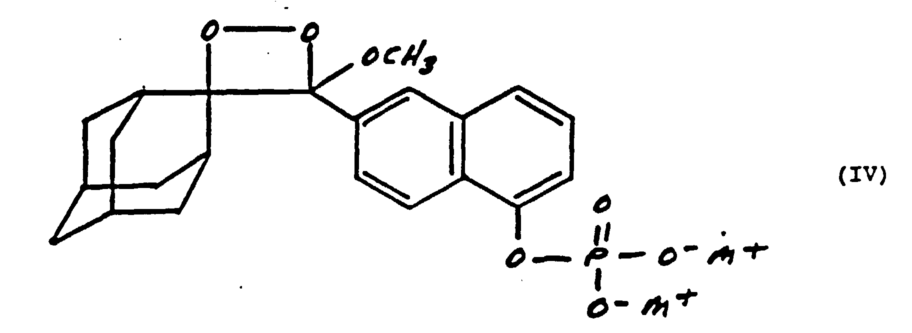 the (5"-phosphoryloxy)naphth-2'-yl isomer, and 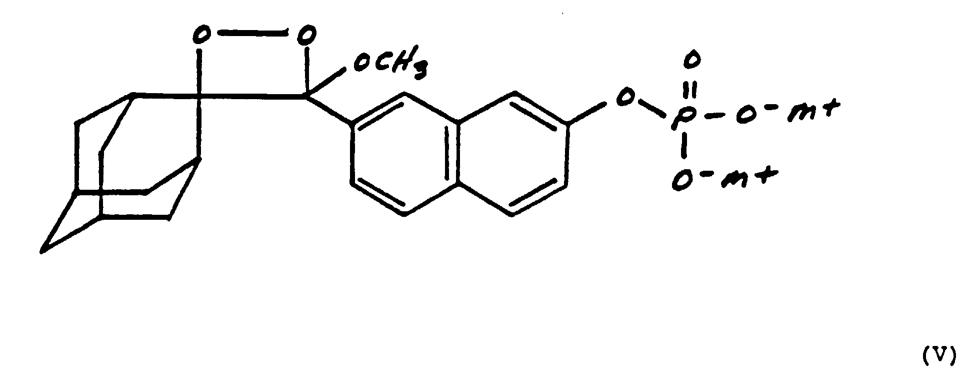 the (7"-phosphoryloxy)naphth-2'-yl isomer,
wherein M+ is as described for formula II above.

The improved chemiluminescent 1,2-dioxetanes of this invention can be synthesized in known manner, e.g., by a reaction scheme involving first the synthesis of an olefin represented by the general formula: 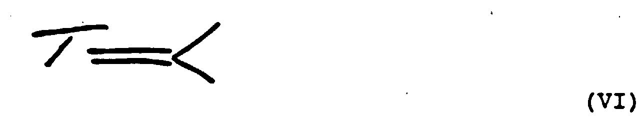 e.g., an enol ether such as: 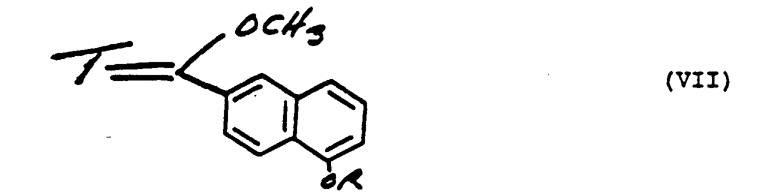 wherein T and R are as described for formula I above, using a Wittig reaction or the methods described in the aforementioned copending Edwards application. Incorporation of a labile ring substituent on the fused polycyclic ring and photochemical or chemical conversion to the corresponding 1,2-dioxetane can then be accomplished as described in the aforementioned copending Bronstein and Edwards applications.

Key intermediates in the synthesis of the improved naphthalene residue-containing chemiluminescent 1,2-dioxetanes of this invention by methods such as those described in the aforementioned copending Edwards application are, first of all, naphthalene residue-containing fluorophoric compounds and having at least an ether substituent, which can be an -OR group as defined above, e.g., an alkoxy or silyloxy group such as a methoxy or t-butyldimethylsilyloxy group, and a halo substituent, i.e., a chlorine, bromine or iodine atom, with the total number of ring carbon atoms separating these two substituents, including the carbon atoms to which they are attached, being an odd whole number. Such intermediates include, for example, naphthalene residue-containing fluorophoric compounds such as:
2-chloro-7-methoxynaphthalene,
2-bromo-7-methoxynaphthalene,
2-iodo-7-methoxynaphthalene,
1-chloro-8-methoxynaphthalene,
1-bromo-8-methoxynaphthalene,
1-iodo-8-methoxynaphthalene,
2-chloro-5-methoxynaphthalene,
2-bromo-5-methoxynaphthalene,
2-iodo-5-methoxynaphthalene,
1-chloro-6-methoxynaphthalene,
1-bromo-6-methoxynaphthalene,
1-iodo-6-methoxynaphthalene,
and the like.

These iodo-substituted ethers can be prepared from the corresponding ether triazenes in the manner described in Example I, infra, using an alkali metal halide such as potassium iodide and an acidic resin. The other halo-substituted ethers can be prepared from the corresponding diazonium salts using Ca₂Cl₂/HCl or CuBr/HBr, or by chlorination or bromination and subsequent oxidation of various methoxy 1-or 2-tetralones, as described in Example VIII, infra.

Also key to such synthetic methods are the next two intermediates in the sequence given, inter alia, in Example I, infra, i.e., the alcohols and ketones having the general formulas: 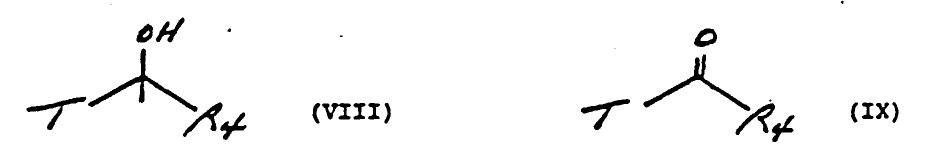 wherein T is as described for formula I above and R₄ is the ether substituted residue of the ether and halogen substituted naphthalene residue-containing compound, in which residue the total number of carbon atoms separating the ring carbon atom to which the ether substituent is attached and the ring carbon atom through which this residue, R₄, is attached to the remainder of the alcohol or ketone molecule (the ring carbon atom to which the precursor intermediate's halogen substituent was attached), including the carbon atoms at the points of attachment, is, once again, an odd whole number. Such alcohol and ketone intermediates include, for example:
7-methoxynaphth-2-yladamant-2'-yl alcohol,
8-methoxynaphth-2-yladamant-1'-yl alcohol,
5-methoxynaphth-2-yladamant-2'-yl alcohol,
6-methoxynaphth-2-yladamant-1'-yl alcohol,
7-methoxynaphth-2-yladamant-2'-yl ketone,
8-methoxynaphth-2-yladamant-1'-yl ketone,
5-methoxynaphth-2-yl-adamant-2'-yl ketone,
6-methoxynaphth-2-yl-adamant-1'-yl ketone,
and the like.

As will be apparent to those skilled in the art, the odd patterned halo, hydroxy fused naphthalene compounds, analogous to the above-listed haloethers and obtainable from these haloethers by cleavage with HBr in acetic acid or by other known methods, are also useful precursors of the improved chemiluminescent 1,2-dioxetanes of this invention. For example, the synthetic method given, inter alia, in Example IX infra can be modified by reacting a 6-halo-1-naphthol, e.g., 6-bromo-1-naphthol, directly with two equivalents of n-butyllithium to generate a dianion which can subsequently be reacted with one equivalent of adamantane-2-carboxaldehyde to generate an alcohol of general formula VIII above, where R₄ is a hydroxy rather than an ether substituted residue. Such hydroxy-and halo-substituted intermediates include, for example:
2-chloro-7-hydroxynaphthalene,
2-bromo-7-hydroxynaphthalene,
2-iodo-7-hydroxynaphthalene,
1-chloro-8-hydroxynaphthalene,
1-bromo-8-hydroxynaphthalene,
1-iodo-8-hydroxynaphthalene,
2-chloro-5-hydroxynaphthalene,
2-bromo-5-hydroxynaphthalene,
2-iodo-5-hydroxynaphthalene,
1-chloro-6-hydroxynaphthalene,
1-bromo-6-hydroxynaphthalene,
1-iodo-6-hydroxynaphthalene,
and the like.

The improved chemiluminescent 1,2-dioxetanes of this invention are particularly useful as the means of identifying or quantifying an analyte or several analytes using otherwise art-recognized immunoassays, such as those employed to detect an enzyme or a member of a specific binding pair, e.g., an antigen-antibody pair or a nucleic acid paired with a probe capable of binding to all or a portion of the nucleic acid. Such assays include immunoassays used to detect a hormone such as β-HCG, thyroid stimulating hormone (TSH), follicle stimulating hormone (FSH), HLH or the like, cell surface receptor assays, and nucleic acid probe assays used to detect viruses, e.g., HIV or HTLV III, Herpes simplex virus (HSV), human papiloma virus (HPV), and cytomegalovirus (CMV), or bacteria, e.g., E.Coli., and histocompatibility assays; for typical assay protocols see working examples X and XI, infra, as well as the aforementioned copending Bronstein and Bronstein et al applications. The improved chemiluminescent 1,2-dioxetanes of this invention can also be used in assays for a chemical analyte, such as potassium or sodium ions, or in assays for substances such as cholesterol or glucose in which the analyte is caused to decompose, for example using an enzyme such, as cholesterol oxidase or glucose oxidase, to form a substance, e.g., hydrogen peroxide, capable in combination with another reagent of causing the chemiluminescent compound to decompose.

As noted above, by using two or more of the improved chemiluminescent 1,2-dioxetanes of this invention that each emit light of a different wavelength from the others, or by using one or more of these different colored light-emitting improved chemiluminescent 1,2-dioxetanes with one or more other chemiluminescent compounds which emit light of yet other wavelengths, each of such compounds being structured so as to be decomposable by a different means, a multi-channel assay can be designed in which different cleaving means, and especially two or more different enzymes, attached to or associated with two or more different analytes will, by cleaving different cleavable dioxetane substituents, induce the emission of light of a different wavelength for each analyte being assayed.

3-(2'-Spiroadamantane)-4-methoxy-4-(3'-disodiumphosphoryloxy)phenyl-1,2-dioxetane, for example, when cleaved with an alkaline phosphatase, will emit blue light, 3-(2'-spiroadamantane)-4-methoxy-4-(7"-acetoxy)naphth-2'-yl-1,2-dioxetane, when cleaved with base or a carboxylesterase, will emit green light, and 3-(2'-spiroadamantane)-4-methoxy-4-(5"-β-galactosyloxy)naphth-2'-yl-1,2-dioxetane, when cleaved with a β-galactosidase, will emit orange light. A simultaneous assay for HSV, CMV and HPV can hence be designed using these three chemiluminescent substances to produce light emissions of a different color for each of these three analytes; see Example XI, infra.

Light of various colors emitted when using the improved chemiluminescent 1,2-dioxetanes of this invention singly or in combinations as discussed above to identify or quantify various analytes can also be used to make a permanent record of such emissions on color photographic emulsions or specially sensitized black and white high speed films. Also, these improved chemiluminescent 1,2-dioxetanes can be used to achieve a matched response by detectors: charged coupled devices (CCD's) or silicon photodiodes, for example, having maximum sensitivity for a color other than blue, e.g., green or red. Thus for example, the blue light emitted when 3-(2'-spiroadamantane)-4-methoxy-4-(6"-disodiumphosphoryloxy)naphth-2'-yl-1,2-dioxetane is cleaved by an alkaline phosphatase to detect β-HCG can be changed to green light simply by replacing this phosphoryloxy naphthyl dioxetane with its odd pattern substituted isomer, 3-(2'-spiroadamantane)-4-methoxy-4-(7"-disodiumphosphoryloxy)naphth-2'-yl-1,2-dioxetane. And by using the enhanced intensity chemiluminescent 1,2-dioxetanes of this invention together with a light absorbing/light shifting auxiliary fluorophore/light enhancer substance which absorbs light of one wavelength and in turn emits light of a different wavelength, e.g., a phycobiliprotein (phycobiliproteins are naturally-occurring substances in which a fluorophore is bonded to a protein), such as phycocyanine or phycoallocyanine, that will absorb the green light emitted by 3-(2'-spiroadamantane)-4-methoxy-4-(7"-disodiumphosphoryloxy)naphth-2'-yl-1,2-dioxetane and reemit this light as red light, matched responses by color photographic emulsions that exhibit a poor response to blue light, a better response to green light but the best response to red light can also be achieved.

Besides the phycobiliproteins, other auxiliary fluorophores extraneous to the light-emitting fluorophores produced by the decomposition of the improved chemiluminescent 1,2-dioxetane compounds of this invention that will accept energy, especially light, from these light-emitting fluorophores and in turn emit detectable energy, again preferably light, can be used when practicing this invention. Among such auxiliary fluorophores that can be used, alone or in combination, are the following substances whose residues can be present in known 1,2-dioxetanes and, in certain cases, if substituted with a labile ring substituent in the appropriate odd numbered pattern, in the improved chemiluminescent 1,2-dioxetanes of this invention as well, as fluorescent chromophore groups:
- anthracene and anthracene derivatives, e.g., 9,10-diphenylanthracene, 9-methylanthracene, 9-anthracene carboxaldehyde, anthrylalcohols and 9-phenylanthracene;
- rhodamine and rhodamine derivatives, e.g., rhodols, tetramethyl rhodamine, tetraethyl rhodamine, diphenyldimethyl rhodamine, diphenyldiethyl rhodamine and dinaphthyl rhodamine;
- fluorescein and fluorescein derivatives, e.g., 5-iodoacetamido fluorescein, 6-iodoacetamido fluorescein and fluorescein-5-maleimide;
- coumarin and coumarin derivatives, e.g., 7-dialkylamino-4-methylcoumarin, 4-bromomethyl-7-methoxycoumarin and 4-bromomethyl-7-hydroxy coumarin;
- erythrosin and erythrosin derivatives, e.g., hydroxy erythrosins, erythrosin-5-iodoacetamide and erythrosin-5-maleimide;
- aciridine and aciridine derivatives, e.g., hydroxy aciridines and 9-methyl aciridine;
- pyrene and pyrene derivatives, e.g., N-(1-pyrene) iodoacetamide, hydroxy pyrenes and 1-pyrenemethyl iodoacetate;
- stilbene and stilbene derivatives, e.g., 6,6'-dibromostilbene and hydroxy stilbenes;
- naphthalene and naphthalene derivatives, e.g., 5-dimethylamino naphthalene-1-sulfonic acid and hydroxy naphthalenes;
- nitrobenzoxadiazoles and nitrobenzoxadiazole derivatives, e.g., hydroxy nitrobenzoxadiazoles, 4-chloro-7-nitrobenz-2-oxa-1,3-diazole, 2-(7-nitrobenz-2-oxa-1,3-diazol-4-yl) methylaminoacetaldehyde and 6-(7-nitrobenz-2-oxa-1,3-diazol-4-yl-aminohexanoic acid;
- quinoline and quinoline derivatives, e.g., 6-hydroxyquinoline and 6-aminoquinoline;
- acridine and acridine derivatives, e.g., N-methylacridine and N-phenylacridine;
- acidoacridine and acidoacridine derivatives, e.g., 9-methylacidoacridine and hydroxy-9-methylacidoacridine;
- carbazole and carbazole derivatives, e.g., N-methylcarbazole and hydroxy-N-methylcarbazole;
- fluorescent cyanines, e.g., DCM (a laser dye), hydroxy cyanines, 1,6-diphenyl-1,3,5-hexatriene, 1-(4-dimethyl aminophenyl)-6-phenylhexatriene and the corresponding 1,3-butadienes;
- carbocyanines and carbocyanine derivatives, e.g., phenylcarbocyanine and hydroxy carbocyanines;
- pyridinium salts, e.g., 4-(4-dialkyldiaminostyryl)N-methyl pyridinium iodate and hydroxy-substituted pyridinium salts;
- oxonols; and
- resorofins and hydroxy resorofins.

When such auxiliary fluorophores are bonded to a chemiluminescent compound, they are preferably bonded to the portion of the chemiluminescent compound that, upon decomposition, forms a fragment containing the fluorophore portion of the chemiluminescent compound's molecule. In this way energy transfer is enhanced due to the two fluorophores being in close proximity to one another and by beneficial spatial arrangements provided by the rigidity of the microenvironment. Auxiliary fluorophores that are insoluble or partially insoluble in aqueous medium can be solubilized by first grafting them onto solubilizing molecules, e.g., water soluble oligomer or polymer molecules.

And, in all cases, enhancement of the intensity of the light emitted by decomposition of the improved chemiluminescent 1,2-dioxetane compounds of this invention in aqueous media can be achieved by the methods disclosed in the aforementioned Voyta et al application.

The invention provides a method for generating light which comprises:
(a) providing a chemiluminescent 1,2-dioxetane compound according to the invention; and
(b) decomposing the 1,2-dioxetane compound by cleaving, e.g. by an enzyme, the labile ring substituent's bond.

The invention also provides a process for determining the presence, concentration or structure of an analyte, which process comprises detecting light released by the decomposition of a chemiluminescent compound, wherein there is used at least one chemiluminescent 1,2-dioxetane compound according to the invention. This process may be carried out as a step in an immunoassay, e.g. for the detection of a specific binding pair comprising an antigen and an antibody. An enzyme or the chemiluminescent 1,2-dioxetane compound may be used as label in the assay. The immunoassay may be used e.g. for the detection of an enzyme, or a hormone.

The determination of analyte may be carried out as a step in a chemical assay, e.g. chemical assay for the detection of a chemical substance, such as glucose or cholesterol, which, during the assay, is caused to decompose to form a substance capable of causing the chemiluminescent 1,2-dioxetane compound to decompose.

The process for the determination of analyte carried out may be a nucleic acid probe assay, e.g. for the detection of a virus, or a histocompatibility assay, or used as a technique for studying the microstructure of a macromolecule.

The process for the determination of analyte according to the invention may be a multi-channel assay carried out in the presence of at least two of the chemiluminescent 1,2-dioxetane compounds according to the invention as substrates, each of which upon decomposition emits light of a different wavelength from the other(s) and each of which has a labile ring substituent cleavable by a different means from the other(s). Alternatively the process for the determination of analyte according to the invention may be a multi-channel assay carried out in the presence of at least one of the chemiluminescent 1,2-dioxetane compounds according to the invention and at least one other chemiluminescent compound as substrates, each of which chemiluminescent 1,2-dioxetane compounds and other chemiluminescent compounds emit light of a different wavelength from the other(s) and each of which is cleavable by different means from the other(s) to produce a light-emitting fluorophore moiety, such as an immunoassay for the quantitation of human chorionic gonadotropin and human luteinizing hormone contained in a single sample and carried out in the presence of 3-(2'-spiroadamantane)-4-methoxy-4-(3'-phosphoryloxy)phenyl-1,2-dioxetane disodium salt and 3-(2'-spiroadamantane)-4-methoxy-4-(7"-acetoxy)naphth-2'-yl-1,2-dioxetane as the chemiluminescent substrates, where the phosphoryloxyphenyl dioxetane chemiluminescent substrate may be cleaved by alkaline phosphatase conjugated to mouse monoclonal anti-β-HCG and the acetoxynaphthyl dioxetane chemiluminescent substrate cleaved by carboxylesterase conjugated to mouse monoclonal anti-HLH, or such as a nucleic acid probe assay for the quantitation of herpes simplex virus, cytomegalovirus and human papiloma virus contained in a single sample and carried out in the presence of 3-(2'-spiroadamantane)-4-methoxy-4-(3'-phosphoryloxy)phenyl-1,2-dioxetane disodium salt, 3-(2'-spiroadamantane)-4-methoxy-4-(7"-acetoxy)naphth-2'-yl-1,2-dioxetane and 3-(2'-spiroadamantane) -4-methoxy-4- (5"-β-galactosyloxy)naphth-2'-yl-1,2-dioxetane as the chemiluminescent substrates, where phosphoryloxyphenyl dioxetane chemiluminescent substrate may be cleaved by a DNA probe for HSV labelled with alkaline phosphatase, the acetoxynaphthyl dioxetane chemiluminescent substrate cleaved by a DNA probe for CMV labelled with carboxylesterase and the β-galactosyloxynaphthyl dioxetane chemiluminescent substrate cleaved by a DNA probe for HPV labelled with β-galactosidase.

In order that those skilled in the art can more fully understand this invention, the following examples are set forth. These examples are given solely for purposes of illustration, and should not be considered as expressing limitations unless so set forth in the appended claims. All parts and percentages are by weight, unless otherwise stated.

### EXAMPLE 1

### [Synthesis of 3-(2'-spiroadamantane)-4-methoxy-4-(7"-acetoxy)naphth-2'-yl-1,2-dioxetane]

2-Amino-7-hydroxynaphthalene. To a solution of 2,7-dihydroxynaphthalene (27.56 g, 0.172 mol) in 30% NH₄OH (200 ml) was added NaHSO₃ (19.03 g, 0.183 mol) at 0°C with stirring. The Bucherer reaction was then carried out by heating the solution in a steel bomb at 150°C for 4.5 hours, giving both mono- and diaminonaphthalenes in a 1:1 ratio. Subsequently, the cooled product mixture was dissolved in EtOAc/CH₃CN (ethyl acetate/methyl cyanide), partitioned with 1M NaOH, followed by washing the organic layer with NaOH until the washes were clear. The combined NaOH layers were acidified to pH 1, partitioned with minimal EtOAc to remove traces of starting material and then carefully neutralized to pH 7 with NaOH pellets. Upon partitioning the aqueous layer with EtOAc, the organic layer was dried over Na₂SO₄ and evaporated to yield 12.49 g (45.7%) of 2-amino-7-hydroxynaphthalene as light tan crystals, m.p. 186-188°C.

IR (Nujol, cm⁻¹): 3401, 3323, 3313, 1629, 1512, 1301, 1216, 883, 831

2-Amino-7-methoxynaphthalene. 2-Amino-7-hydroxynaphthalene (18.6 g, 0.117 mol) was added in portions to hexane-rinsed NaH (60%, 5.1 g, 0.127 mol) in dry DMF (dimethylformamide; 100 ml) at 0°C. The solution was stirred under argon for 10 minutes until hydrogen evolution had ceased, whereupon dimethyl sulfate (11 ml, 0.116 mol) was added dropwise via syringe at 0°C until O-methylation was complete by TLC monitoring. The reaction was carefully quenched with 6N HCl to pH 1, and partitioned between ether and water, thus separating the naphthylamine hydrochloride from organic impurities. After washing the ether layer with 6N HCl, the aqueous layers were combined, neutralized with NaOH pellets to pH 8 and partitioned with EtOAc to recover the free amine in the organic layer. The basic aqueous layer was washed twice with EtOAc, and the combined EtOAc layers were dried over Na₂SO₄ and evaporated. Upon removal of DMF under high vacuum, the product mixture crystallized. Crystals of 2-amino-7-methoxynaphthalene (19.4 g, 96.5%) were collected from MeOH, m.p. 157°C.

IR (Nujol, cm⁻¹): 3403, 3318, 1628, 1511, 1213, 1028, 871, 826

NMR (CDCl₃, ppm): 3.54 (2H, br s); 3.90 (3H, s); 6.73-7.00 (4H, m); 7.54-7.60 (2H)

2-Iodo-7-methoxynaphthalene. Diazotization of 2-amino-7-methoxynaphthalene (17.02 g, 0.098 mol) in 20% aqueous HCl (81.4 ml, 0.197 mol HCl), proceeded smoothly by adding NaNO₂ (7.11 g, 0.103 mol) in 65 ml H₂O dropwise at 0°C. The reaction was stirred for 1 hour, adding a small amount of EtOAc to reduce foaming as needed. To form the triazene, a solution of K₂CO₃ (20.41 g, 0.148 mol), diethylamine (15.3 ml, 0.148 mol) and H₂O (60 ml) was added dropwise to the cooled diazonium salt. After adding ethyl ether (30 ml) to dissolve the newly formed naphthalene triazene, followed by stirring for 35 minutes at low temperature, the reaction solution was poured into a separatory funnel and the aqueous layer was drawn off. The ether layer was washed with H₂O, dried over Na₂SO₄ and evaporated under reduced pressure with low heat, giving an orange oil. The crude diethyltriazene was used immediately without purification.

2-(3',3'-Diethyl)triazo-7-methoxynaphthalene, dissolved in 120 ml dry CH₃CN, was added dropwise (1 drop/4 seconds) to a slurry of ground XI (49.12 g, 0.296 mol), Amberlyst XN-1010 acidic resin (Aldrich Chemical Co.;- 37.3 g), and ground molecular sieves in dry DMF/CH₃CN (50 ml/150 ml) at 70°C overnight. Upon complete conversion of the triazene to the iodide, the cooled solution was carefully decanted into a separatory funnel, rinsing the resin with two EtOAc washes. The organic material was partitioned between EtOAc and H₂O, collecting the EtOAC layer, which was dried over Na₂SO₄ and evaporated.

To purify the iodonaphthalene, the crude reaction mixture was dissolved in CH₃CN and partitioned with hexanes until all desired iodide had been removed from the CH₃CN layer. The hexanes solution was evaporated to yield, upon addition of petroleum ether, acrid orange crystals of 2-iodo-7-methoxynaphthalene (10.36 g, 33.1% m.p. 116°C). [Literature reference: J.Org.Chem., (1983) 48, 4396.]

IR (CHCl₃, cm⁻¹): 3005, 1625, 1504, 1458, 1394, 1359, 1250, 1176, 1126, 1031, 958, 916, 886, 841, 631

NMR (CDCl₃, ppm): 3.82 (3H, s); 6.91 (1H, d, J=2.44 Hz); 7.05 (1H, dd, J=8.9, 2.5 Hz); 7.39 (1H, d, J=8.47 Hz); 7.05 (1H, dd, J=8.9, 2.5 Hz); 7.39 (1H, d, J=8.47 Hz); 7.47 (1H, d, J=8.46 Hz); 7.59 (1H, d, J=9.0 Hz); 8.04 (1H, s)

By using halogenating agents such as Cu₂Cl₂/HCl and CuBr/HBr
in combination with the diazonium salt intermediate instead of the triazene, 2-chloro-7-methoxynaphthalene and 2-bromo-7-methoxynaphthalene are obtained.

7-Methoxynaphth-2-yladamant-2'-yl alcohol. Lithiation of 2-iodo-7-methoxynaphthalene (508.1 mg, 1.79 mmol) in 5 ml anhydrous ether proceeded cleanly upon addition of n-BuLi (n-butyllithium; 1.6M, 1.2 ml) at 0°C under argon. Dropwise addition of adamantane-2-carboxaldehyde, dissolved in anhydrous ether (2 ml), to the naphthyllithium solution immediately yielded the coupled lithium alkoxide. The reaction was quenched by partitioning between EtOAc/H₂O, washing the aqueous layer twice with EtOAc to completely recover the polar alcohols. The combined organic layers were dried over Na₂SO₄, evaporated and the residual oil was chromatographed on silica gel, eluting with 20% EtOAc/hexanes, to give an off-white crystalline alcohol mixture (437.9 mg, 76%).

IR, less polar alcohol (CHCl₃, cm⁻¹): 3601, 3005, 2905, 2846, 1632, 1607, 1515, 1465, 1391, 1265, 1175, 1125, 1034, 895, 845, 705

IR, polar alcohol (CHCl₃, cm⁻¹): 3589, 3005, 2905, 2849, 1631, 1609, 1515, 1465, 1393, 1259, 1137, 1129, 1035, 845, 805, 680

7-Methoxynaphth-2-yladamant-2'-yl ketone. The 7-methoxynaphth-2-yladamant-2'-yl epimeric alcohols (238 mg, 0.739 mmol) in 6 ml acetone were oxidized by dropwise addition of Jones reagent at 0°C until analytical TLC indicated complete conversion to the ketone. The reaction mixture was partitioned between EtOAc and H₂O and the aqueous layer was washed three times with EtOAc. The combined EtOAc layers were washed with minimal saturated NaHCO₃ solution, dried through a Na₂SO₄ column, and evaporated to a light yellow oil. The crude ketone was used without purification. A small amount was chromatographed (5% EtOAc/hexanes) and crystallized from acetone as a fine white powder, m.p. 116°C.

IR (CHCl₃, cm⁻¹): 2908, 2846, 1674 (C=O), 1629, 1605, 1466, 1272, 1257, 1179, 1132, 1126, 1105, 1035, 844

NMR (CDCl₃, ppm): 1.59-2.13 (12H); 2.39 (2H, s); 3.59 (1H, s); 3.94 (3H, s); 7.23 (1H, s); 7.245 (1H, d, J=3.61 Hz); 7.74 (1H, d, J=3.17 Hz); 7.76 (1H, d, J=8.30 Hz); 7.80 (1H, d, J=8.36 Hz); 8.24 (1H, s)

Methoxy(7-methoxynaphth-2-yl)methylene adamantane. Anion formation of 7-methoxynaphth-2-yladamant-2'-yl ketone (236 mg, 0.739 mmol) occurred at 50°C in the presence of potassium t-butoxide (150.6 mg, 1.34 mmol) in 3.5 ml of DMSO under argon. After stirring for 5 minutes, addition of dimethyl sulfate (120 µl, 1.27 mmol) gave O-methylation, with a trace of ketone remaining. The cooled solution was partitioned with EtOAc/H₂O and the EtOAc layer was dried through a Na₂SO₄ column and evaporated. A small amount of the residual oil was separated on an analytical TLC plate (silica gel) for spectroscopic characterization. The crude enol ether was used in the next step without purification.

IR (CHCl₃, cm⁻¹): 2905, 2843, 1627, 1604, 1510, 1463, 1214, 1124, 1091, 1033, 848

Methoxy(7-acetoxynaphth-2-yl)methylene adamantane. Crude methoxy(7-methoxynaphth-2-yl)methylene adamantane (247 mg, 0.739 mmol) was added to sodium ethylthiolate (62 mg, 0.743 mmol) in dry DMF (4 ml) and the solution was refluxed under argon for 4 hours deprotecting the aromatic methoxy moiety to the naphthol. Excess sodium ethanethiolate (122 mg, 1.45 mmol) was added in two portions during the reflux to push the demethylation to completion. The naphthol was worked up by partitioning the cooled solution between EtOAc and minimal H₂O. Traces of naphthol in the aqueous layer were recovered with two EtOAc washes. The EtOAc layers were dried and evaporated. The crude naphthol was immediately acylated upon dissolution in acetic anhydride (1 ml) and pyridine (2 ml) with gentle warming to 50°C. After evaporation, the reaction mixture was chromatographed on a silica gel column prewashed with 10% EtOAc/hexanes containing 1% triethylamine, eluting with the same EtOAc/hexanes/TEA solvent mixture. The four-step reaction sequence--oxidation, enol ether formation, aromatic demethylation and acylation--gave 69.3 mg (25.8%) of methoxy(7-acetoxynaphth-2-yl)methylene adamantane, m.p. 125°C.

IR (CH₂Cl₂, cm⁻¹): 2897, 2847, 1757, 1629, 1606, 1508, 1448, 1372, 1212, 1198, 1157, 1104, 1092, 1083, 1017, 968, 919, 849

NMR (CDCl₃, ppm): 1.8-2.0 (13H); 2.38 (3H, s); 2.69 (1H, br s); 3.3 (3H, s); 7.21 (1H, dd, J=9.0, 2.5 Hz); 7.44 (1H, dd, J=8.5, 1.5 Hz); 7.54 (1H, d, J=1.6 Hz); 7.71 (1H,s); 7.82 (1H, d, J=8.0 Hz); 7.84 (1H, d, J=8.9 Hz)

3-(2'-spiroadamantane)-4-methoxy-4-(7'-acetoxy)naphth-2'-yl-1.2-dioxetane. A solution of methoxy(7-acetoxynaphth-2-yl)-methylene admanantane (29.0 mg, 0.080 mmol) and methylene blue adsorbed on silica gel (97.9 mg, 2.6 mg dye/g SiO₂) in methylene chloride (12 ml) was irradiated with a 250 Watt high pressure sodium lamp at 10°C while passing a stream of oxygen through the solution. A 5 mil thick piece of Kapton polyimide film (DuPont) placed between the lamp and the reaction mixture filtered unwanted UV radiation. TLC and UV monitoring showed complete dioxetane formation after irradiating for 25 minutes. The sensitizer was removed by filtration, the solvent was evaporated and the resulting crude oil was chromatographed on silica gel, eluting with 10% THF (tetrahydrofuran) /hexanes to give 26.0 mg (82%) of the 7"-acetoxynaphth-2'-yl dioxetane.

Starting with 1,8-dihydroxynaphthalene and proceeding through the same synthesis as in Example I gives:
1-amino-8-hydroxynaphthalene,
1-amino-8-methoxynaphthalene,
1-iodo-8-methoxynaphthalene,
8-methoxynaphth-2-yladamant-1'-yl alcohols,
8-methoxynaphth-2-yladamant-1'-yl ketone,
methoxy(8-methoxynaphth-1-yl)methylene adamantane,
methoxy(8-acetoxynaphth-1-yl)methylene adamantane, and
3-(2'-spiroadamantane)-4-methoxy-4-(8"-acetoxy)naphth-1'-yl-1,2-dioxetane.
And by synthesizing:
methoxy(7-disodiumphosphoryloxynaphth-2-yl)-methylene adamantane, and
methoxy(8-disodiumphosphoryloxynaphth-1-yl)-methylene adamantane,
by phosphorylating the corresponding methoxynaphthyl compounds in the manner disclosed in the aforementioned copending Edwards application and then photooxygenating these phosphate esters as described in Example I above, the corresponding 1,2-dioxetanes:
3-(2'-spiroadamantane)-4-methoxy-4-(7"-disodiumphosphoryloxy)naphth-2'-yl-1,2-dioxetane, and
3-(2'-spiroadamantane)-4-methoxy-4-(8"-disodiumphosphoryloxy)naphth-1'-yl-1,2-dioxetane,
are obtained.

### EXAMPLE II

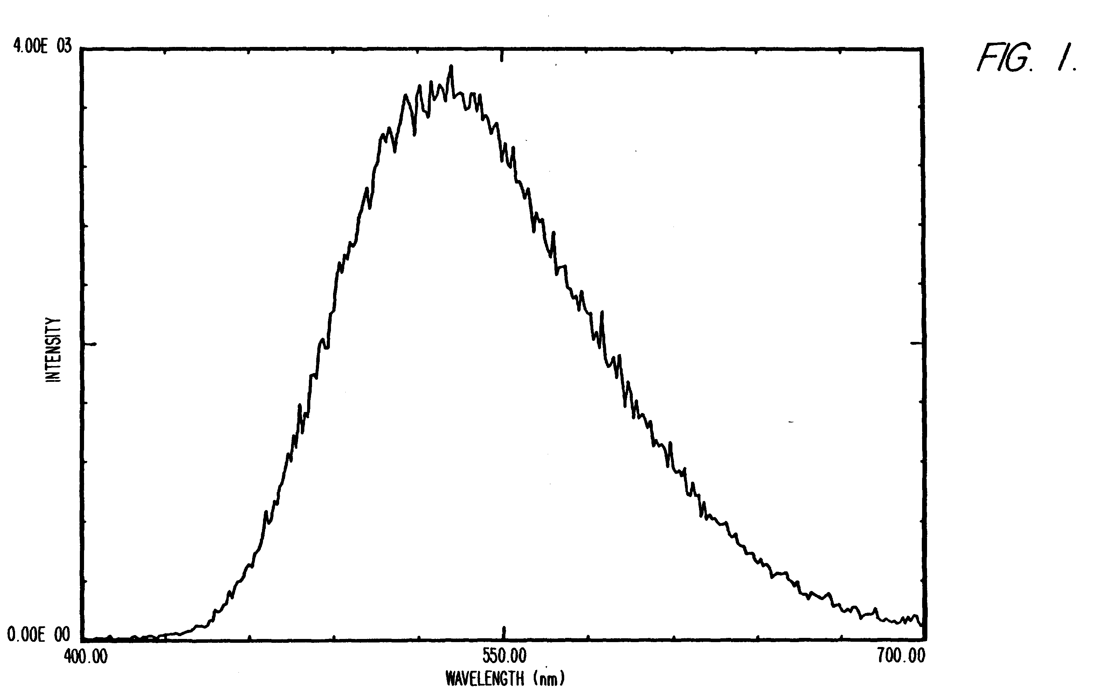

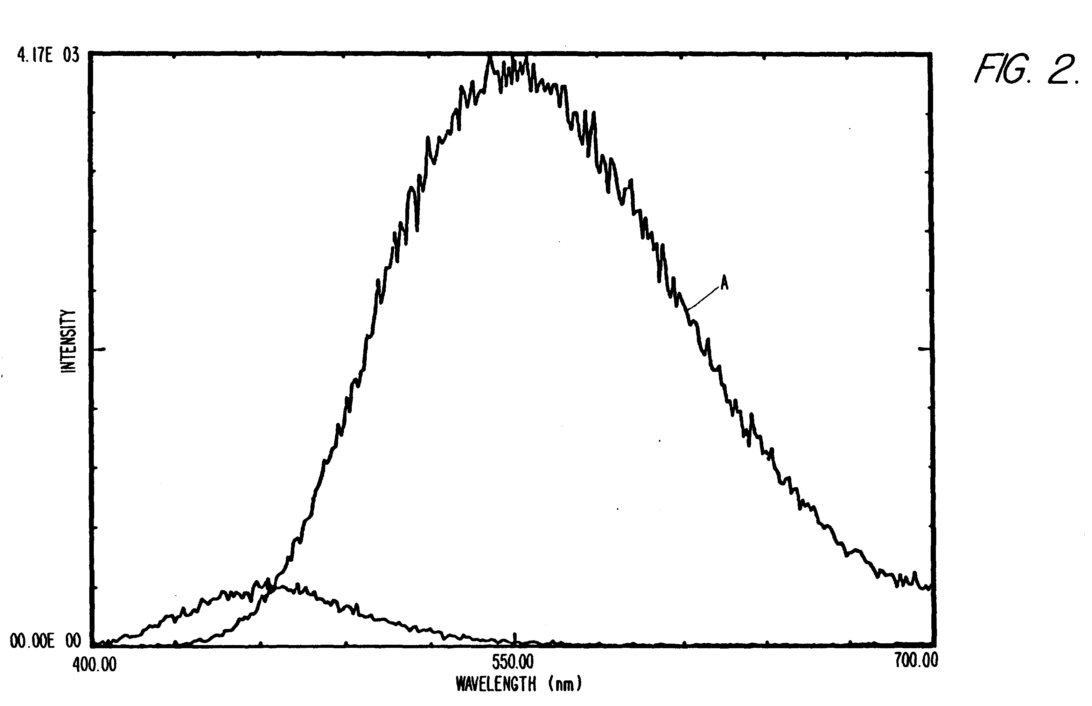

Forty µl of a 5 x 10⁻³M acetonitrile solution of chromatographed 3-(2'-spiroadamantane)-4-methoxy-4-(7"-acetoxy)naphth-2'-yl-1,2-dioxetane were dissolved in 2 ml of phosphate buffer (pH=8) containing 0.1% bovine serum albumin (BSA) in a cuvett. Eight µl of a carboxylesterase suspension (Sigma No. E3128) were added to the cuvett and the resulting light emission was recorded on a Spex Fluorolog Fluorimeter by accumulating 5 successive scans (400-700 nm.). A plot of this emission's intensity vs. wavelength, showing a slight blue shift in the presence of BSA as compared to hydroxide ion-induced decomposition in water (530 nm. v. 555 nm.), is attached as FIG. 1 (compare with FIG. 2, Curve A).

### EXAMPLE III

Methoxy(6-acetoxynaphth-2-yl)methylene adamantane was synthesized as described in the aforementioned copending Edwards application. This compound was then photooxygenated in the same manner as described in Example I above. The resulting 3-(2'-spiroadamantane)-4-methoxy-4-(6"-acetoxy)naphth-2'-yl-1,2-dioxetane, after column chromatography, exhibited the following NMR spectrum (CDCl₃, p.p.m.): 0.95-2.1 (12H, m); 2,25 (lH, s); 2.38 (3H, s); 3.12 (1H, s); 3.28 (3H, s); 7.28-8.15 (6H, m). One hundred µl of a 5 X 10⁻³M solution of this dioxetane in acetonitrile was placed in a cuvett, followed quickly by the addition of 2 ml 75 mM NaOH solution. The slightly cloudy solution was placed in a Spex Fluorolog Fluorometer and light emission accumulated over 5 successive scans from 400 to 700 nm. at room temperature. This experiment was then repeated exactly using a 5 X 10⁻³M acetonitrile solution of the corresponding 2,7-substituted dioxetane as synthesized in Example I. The chemiluminescent emission spectra of the two dioxetanes were plotted simultaneously on intensity vs. wavelength axes. The emission from the 2,7-isomer in this predominately aqueous experiment occurred at 555 nm. while the less intense emission from the 2,6-isomer occurred at 459 nm. in the same medium. This plot is attached as FIG. 2 ("A" is the curve for the 2,7-naphthalene dioxetane isomer).

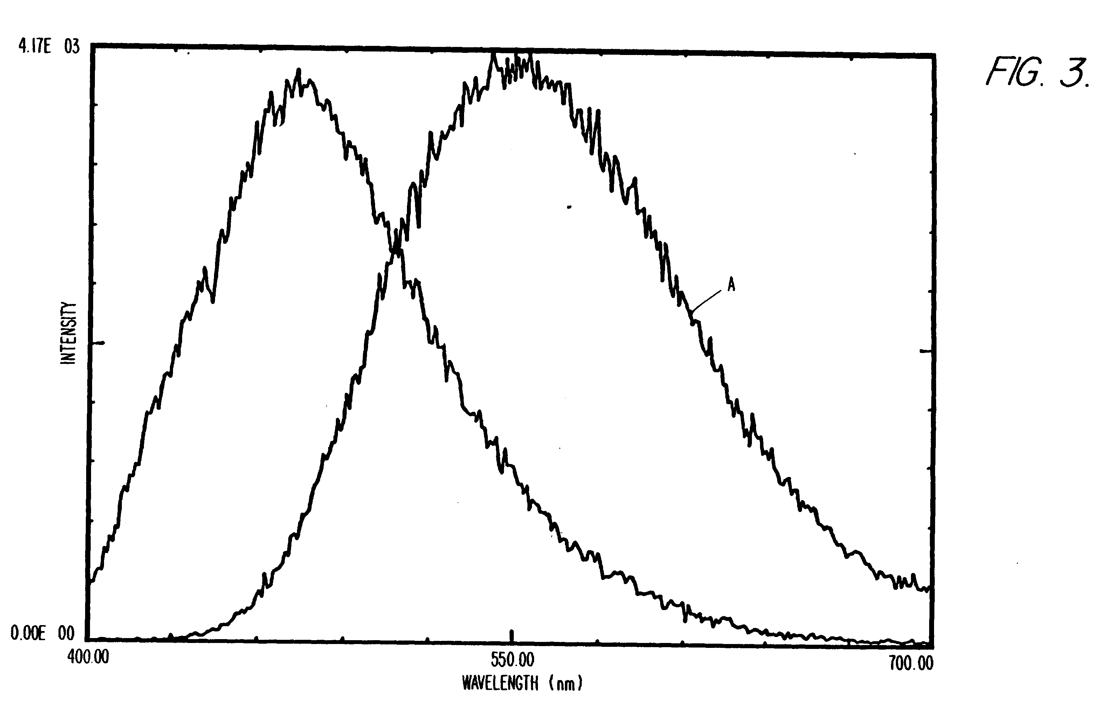

An identical set of experiments was then performed comparing the emission from 3-(2'-spiroadamantane-4-methoxy-4-(7"-acetoxy)naphth-2'-yl-1,2-dioxetane and 3-(2'-spiroadamantane)-4-methoxy-4-(3"-acetoxy)phenyl-1,2-dioxetane (synthesized as described in the aforementioned copending Edwards application) as 5 X 10⁻³ M solutions in CH₃CN. The comparative plot is shown in FIG. 3 ("A" is the curve for the 2,7-naphthalene dioxetane).

The naphthalene based system again emits light at 555 nm., while the acetoxyphenyl dioxetane does so as 473 nm. with similar intensity.

### EXAMPLE IV

One hundred µl of a 5 X 10⁻³ M solution of 3-(2'-spiroadamantane)-4-methoxy-4-(6"-acetoxy)napth-2'-yl-1,2-dioxetane in CH₃CN was placed in a cuvett, followed by the rapid addition of 2 ml 75 mM NaOH in 20% CH₃CN/H₂O. The clear solution was placed in a Spex Fluorolog Fluorometer at room temperature and the resulting light emission recorded by accumulating 5 successive scans (400-700 nm.). This process was repeated for an equimolar CH₃CN solution of 3-(2'-spiroadamantane)-4-methoxy-4-(7"-acetoxy)napth-2'-yl-1,2-dioxetane. FIG. 4 shows the intensity vs. wavelength plot for the chemiluminescence produced via saponification in each isomer. The 2,6-isomer displays its emission maximum at 436 nm. while the more intense emission of the 2,7-isomer occurs at 543 nm. in the mixed solvent ("A" is the curve for the 2,7-naphthalene dioxetane).

### EXAMPLE V

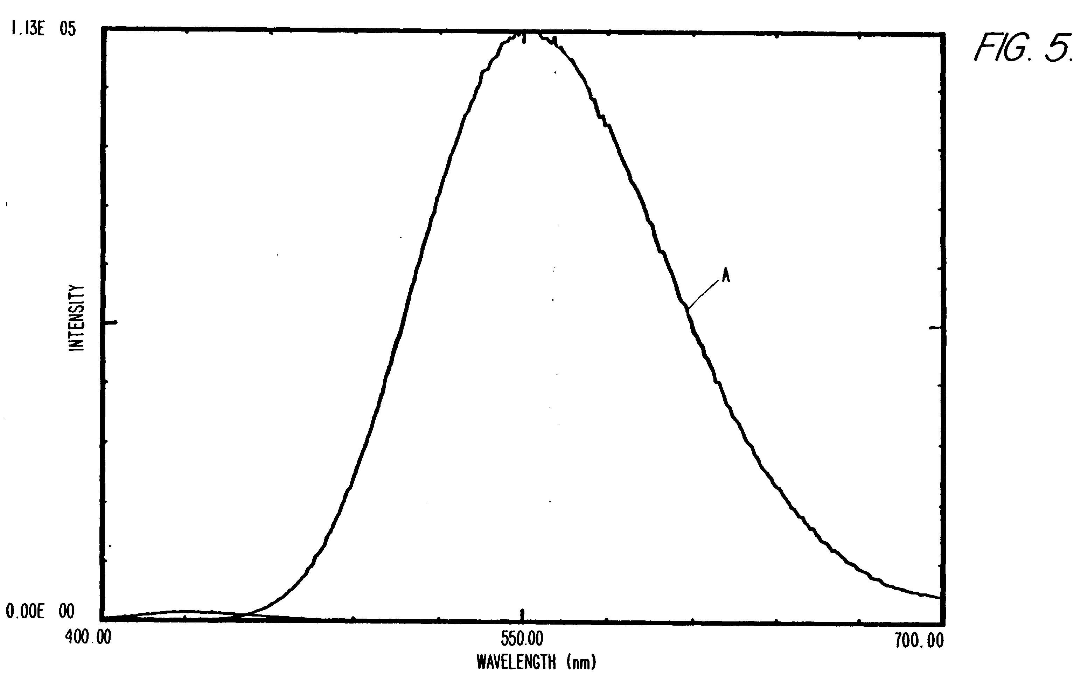

The procedure of Example IV was followed exactly, except that 2 ml of 75 mM NaOMe (Aldrich Chemical Co.) in acetonitrile containing the minimum methanol required to maintain homogeneity was used as the non-aqueous solvent. The emission plots obtained for the two isomeric acetoxy naphthalene dioxetanes (2,6 and 2,7) under these non-aqueous conditions are shown in FIG. 5. The 2,7 isomer emits light at 552 nm. while the 2,6 isomer emits at 430 nm. ("A" is the curve for the 2,7-naphthalene dioxetane).

### EXAMPLE VI

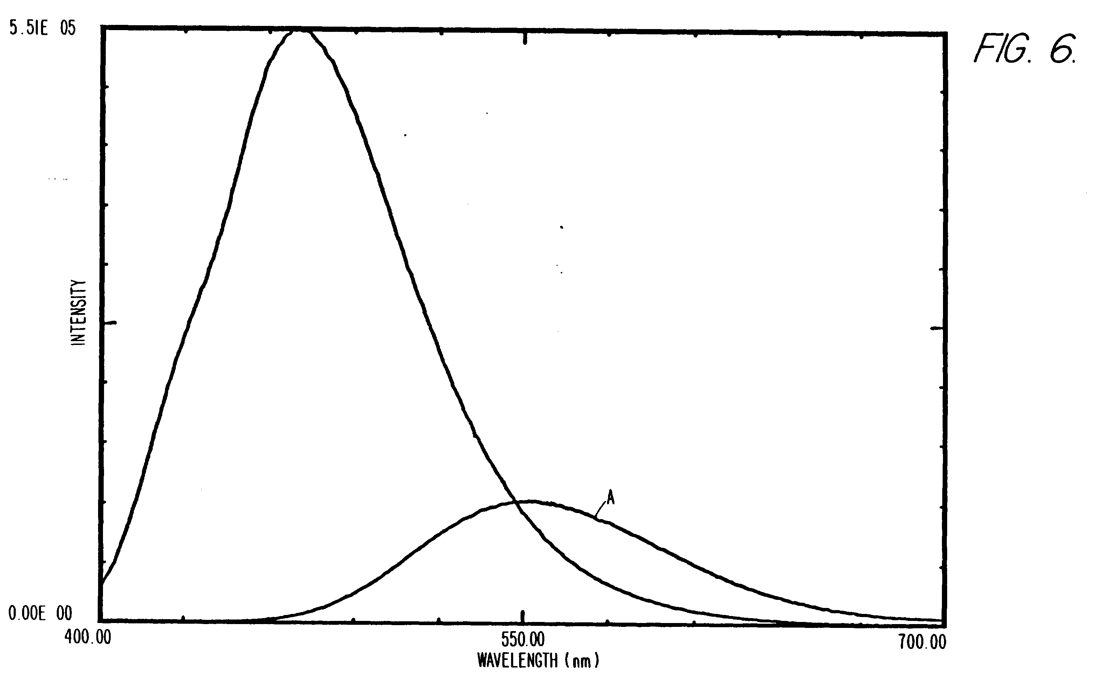

Following the procedure of Example V, 100 µl of 5 X 10⁻³M solutions of 3-(2'-spiroadamantane)-4-methoxy-4-(3"-acetoxy)phenyl -1,2-dioxetane and 3-(2'-spiroadamantane)-4-methoxy-4-(7"-acetoxy)naphth-2'-yl-1,2-dioxetane were independently saponified in the fluorometer with 2 ml 75 mM NaOMe (Aldrich) at room temperature. After 5 scans, the light emission from 400 to 700 nm. in each case was plotted, and is attached as FIG. 6. The 2,7-naphthalene dioxetane (Curve "A") still emits in the green region of the visible spectrum (554 nm.).

### EXAMPLE VII

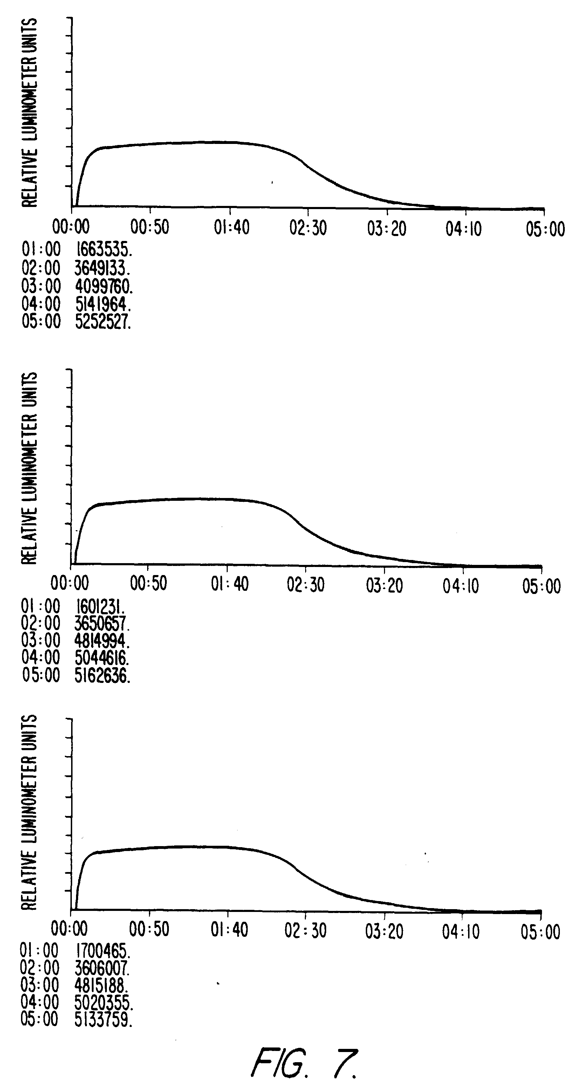

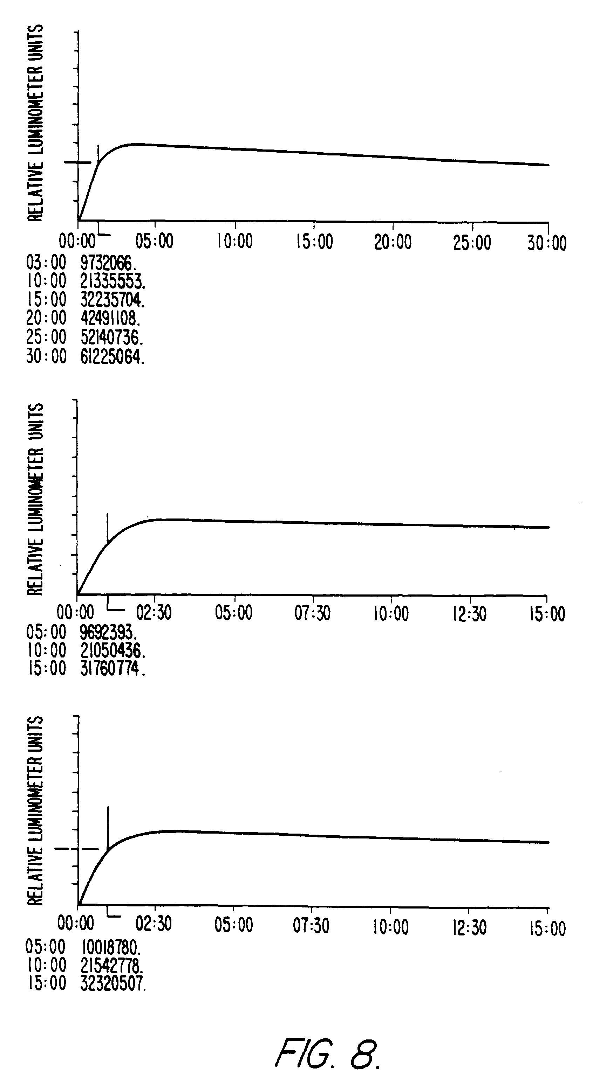

Twenty µl of a 5 x 10⁻³M solution of 3-(2'-spiroadamantane)-4-methoxy-4-(6"-acetoxy)naphth-2'-yl-1,2-dioxetane in CH₃CN was placed in a glass tube inside a Turner Model 20E Luminometer at room temperature. Four hundred fifty µl of a 75 mM solution of NaOH in 20% CH₃CN/80% H₂O was injected manually. Light emission essentially ceased after 3.5 minutes (FIG. 7). The experiment was performed in triplicate and the procedure was then repeated for an equimolar solution of the isomeric 2,7-acetoxynaphthalene dioxetane. In the latter case, the longer duration (beyond 30 minutes) and the larger total integral of light energy are displayed in FIG. 8. The extended glow of the 2,7-substituted dioxetane provides greater sensitivity, simplicity of light detection and flexibility of design in immunoassays and DNA probe technology.

### EXAMPLE VIII

### [Synthesis of 3-(2'-spiroadamantane)-4-methoxy-4-(6"-acetoxy)naphth-1'-yl-1,2-Dioxetane]

1-Amino-6-hydroxynaphthalene. Sodium bisulfite (17.96 g, 0.173 mol) was added in portions to a solution of 1,6-dihydroxynaphthalene (25.14 g, 0.157 mol) in 30% NH₄OH (200 ml) at 0°C with stirring. The reaction mixture was then heated in a steel bomb at 150°C for 3 hours to induce α-amination. After cooling to room temperature, the solution was acidified with concentrated HCl and partitioned with EtOAc/H₂O, forcing the naphthylamine hydrochloride into the aqueous layer. The aqueous layer was neutralized with NaOH pellets to free the desired amine, partitioned with EtOAc, washing the aqueous layer once, and the combined EtOAc layers were dried over Na₂SO₄, decanted and evaporated, yielding dark brown crystals of 1-amino-6-hydroxynaphthalene [16.75 g, 66.3%, m.p. 180°C, dec.; literature value is m.p. 186.8°C, JACS, 51 (1929) 1766.].

IR (Nujol, cm⁻¹): 3353, 3387, 1600, 1574, 1303, 1271, 1228

1-Amino-6-methoxynaphthalene. 1-Amino-6-hydroxynaphthalene (16.75 g, 0.105 mol) was added in portions to hexane-rinsed NaH (60% 5.13 g, 0.128 mol) in dry DMF (150 ml) at 0°C. The solution was stirred under argon for 10 minutes until hydrogen evolution had ceased, indicating complete sodium naphthoate formation. O-methylation proceeded via dropwise addition of dimethyl sulfate (10 ml, 0.106 mol) at 0°C until TLC showed consumption of all starting naphthol. The reaction was carefully quenched with concentrated HCl to pH 1 and partitioned between ether and H₂O. After washing the ether layer with 6N HCl, the combined aqueous layers, containing the desired naphthylamine hydrochloride, were neutralized with NaOH pellets, partitioned with EtOAc and washed twice. The EtOAc layers were dried over Na₂SO₄, concentrated by rotary evaporator and the residue was pumped under high vacuum while warming to remove DMF. The crude product was chromatographed on coarse silica gel, eluting with 20% EtOAc/hexanes. Dissolution of the oil in MeOH/H₂O yielded 16.53 g (90.7%) of crystalline 1-amino-6-methoxynaphthalene, m.p. 64°C (Literature value is m.p. 74°C, but has also been reported as 64°C [J.Org.Chem., 14 (1949) 352).]

IR (CHCl₃, cm⁻¹): 3468, 3445, 3393, 3000, 1707, 1623, 1447, 1362, 1271, 1225

NMR (CDCl₃, ppm): 3.89 (3H, s); 4.05 (2H, br s); 6.62 (1H, d, J=7.03 Hz); 7.11 (2H, m); 7.22 (1H, d); 7.24 (1H, d); 7.69 (1H, d, J=9.96 Hz)

1-Iodo-6-methoxynaphthalene. Diazotiation of 1-amino-6-methoxynaphthalene (16.53 g, 0.096 mol) in an HOAc (acetic acid) concentrated HCl/H₂O mixture (9 ml/24 ml/90 ml) proceeded with dropwise addition of NaNO₂ (7.0 g, 0.101 mol) dissolved in 20 ml of H₂O at 0°C. The reaction mixture was stirred for 1 hour at low temperature and then converted to the N,N-diethyltriazene upon addition of K₂CO₃ (33 g, 0.239 mol, dissolved in 20 ml H₂O and diethylamine (15 ml, 0.145 mol). After adding ethyl ether (50 ml) to dissolve the newly formed naphthalene triazene, followed by stirring for 40 minutes, the reaction solution was partitioned with CH₃CN/EtOAc 1:1. The aqueous phase was drawn off and washed well with CH₃CN/EtOAc. The combined organic layers were dried and evaporated under reduced pressure with low heat to give the crude triazene as an orange oil, which was used immediately, without purification, in the next reaction.

1-(3',3-Diethyl)triazo-6-methoxynaphthalene, dissolved in 120 ml dry CH₃CN, was added dropwise (1 drop/4 sec) to a slurry of ground KI (35 g, 0.211 mol), Amberlyst XN -1010 resin (30 g) and ground molecular sieves in dry DMF/CH₃CN (50 ml/150 ml) at 70°C overnight. When TLC indicated complete iodide formation, the reaction mixture was cooled, decanted into a separatory funnel and the resin was rinsed with three CH₃CN washes. The CH₃CN layers were combined, evaporated to a small volume and partitioned with hexanes as necessary to thoroughly extract the desired iodonaphthalene from the CH₃CN layer. The hexanes solution was evaporated and quickly passed through coarse silica gel, eluting with hexanes, to yield 7.245 g (27%) of 1-iodo-6-methoxynaphthalene as a yellow-orange oil which slowly crystallized upon refrigeration.

IR (CHCl₃, cm⁻¹): 1622, 1593, 1501, 1468, 1372, 1355, 1234, 1171, 1123, 1032, 955, 846, 823, 652

NMR (CDCl₃, ppm): 3.85 (3H, s); 6.99 (1H, d, J=2.55 Hz); 7.02 (1H, m); 7.12 (1H, dd, J=9.28, 2.55 Hz); 7.63 (1H, d, J=8.09 Hz); 7.82 (1H, dd, J=7.32, 1.09 Hz); 7.90 (1H, d, J=9.28 Hz)

6-Methoxynaphth-1-yladamant-1'-yl alcohol. Lithiation of 1-iodo-6-methoxynaphthalene (38 mg, 0.134 mmol) in 1 ml anhydrous ether proceeded cleanly upon addition of n-BuLi (1.6M, 250 µl) at -65°C with subsequent warming to -15°C under an argon atmosphere. Dropwise addition of adamantane-2-carboxaldehyde (41 mg, 0.25 mmol) dissolved in 1 ml anhydrous ether to the naphthylithium solution immediately yielded the coupling product. The reaction was quenched upon partitioning between EtOAc and H₂O, the EtOAc was dried through a Na₂SO₄ column and purified on a silica gel column, eluting with 5-20% EtOAC/hexanes, to yield 5.5 mg of alcohol for spectral characterization.

IR (CHCl₃, cm⁻¹): 3603, 2904, 2847, 1624, 1601, 1512, 1473, 1453, 1434, 1377, 1172, 1044, 1020, 851, 831

6-Methoxynaphthyladamant-2'-yl ketone. The 6-methoxynaphth-1-yladamant-2'-yl alcohols (5.5 mg, 0.017 mmol) were dissolved in 1 ml acetone and oxidized to the aromatic ketone with Jones reagent at 0°C. After partitioning the solution between EtOAc/H₂O and washing the aqueous layer three times with EtOAc, the collected organic layers were dried and evaporated to a film. The crude ketone was purified on analytical TLC, eluting the plate with 10% EtOAc/hexanes, to give 4.4 mg (80%) of the desired ketone.

IR (CHCl₃, cm⁻¹): 3021, 2905, 2845, 1674 (C=O), 1623, 1593, 1508, 1471, 1453, 1433, 1374, 1173, 1095, 1044, 843

NMR (CDCl₃, ppm): 1.48-1.93 (12H); 2.29 (2H, br s); 3.28 (1H, br s); 3.84 (3H, s); 7.07 (1H, d, J=2.66 Hz); 7.12 (1H, dd, J=9.34, 2.6 Hz); 7.33 (1H, m); 7.38 (11h, DD, j=7.11, 1.35 hZ); 772 (1h, D, j=7.87 hZ); 8.09 (1h, D, j=9.27 hZ)

An alternative synthesis of the 1,6-naphthalene regioisomer is described below, resulting in 1-bromo-6-methoxynaphthalene. As described above for 1-iodo-6-methoxynaphthalene, the naphthalene bromide was also coupled with adamantane-2-carboxaldehyde and oxidized to the 6-methoxynaphth-1-yladamant-1'-yl ketone. The nmr data for the naphthyladamantyl ketones, derived independently from the iodo- and bromonaphthalene derivatives are virtually superimposable, thus confirming the regiochemistry of the naphthyl halides as being identical.

1-Bromo-6-methoxynaphthalene. 6-Methoxy-1-tetralone (14.42 g, 0.082 mol) dissolved in EtOAc (100 ml) was added to a solution of freshly made PBr₅ (38.8 g, 0.090 mol) and Na₂SO₃ (5.9 g, 0.047 mol) in 100 ml EtOAc, with stirring, at room temperature. The reaction mixture was gently refluxed until analytical TLC indicated no change in the product mixture 23 hours), at which time the solution was cooled and portioned between EtOAc and H₂O. The EtOAc layer was than dried over Na₂SO₄, decanted and evaporated to yield crude 1-bromo-3,4-dihydro-6-methoxynaphthalene.

The dihydronaphthalene was aromatized by refluxing with p-chloranil (12.76 g, 0.052 mol) and 0.5 ml HOAc in toluene (160 ml) for 68 hours. Upon complete oxidation, the cooled mixture was portioned between EtOAc/CH₃CN 11:1 and 1M NaOH. The organic layer was washed with dilute NaOH twice and the combined aqueous layers were, in turn, washed with EtOAc once. The organic layers were then dried over Na₂SO₄, decanted and evaporated under reduced pressure to a brown oil. Chromatography on coarse silica gel (60-200 mesh), with hexanes as the eluent, gave 10.3 g (53% in 2 steps) of the desired 1-bromo-6-methoxynaphthalene.

IR (CHCl₃, cm⁻¹): 3003, 2958, 2937, 1622, 1594, 1564, 1503, 1468, 1423, 1373, 1359, 1263, 1233, 1170, 1125, 1033, 960, 918, 844, 823, 661

NMR (CDCl₃, ppm): 3.86 (3H, s); 7.05 (1H, d, J=2.44 Hz); 7.15-7.21 (2H, m); 7.57 (1H, d, J=6.59 Hz); 7.60 (1H, d, J=8.11 Hz); 8.06 (1H, d, J-9.28 Hz)

6-Methoxynaphth-1-yladamant-1'-yl alcohol. 1-Bromo-6-methoxynaphthalene (92.3 mg, 0.389 mmol) dissolved in 2 ml anhydrous ether was lithiated upon addition of n-BuLi (1.6M, 365 µl) at -60°C under argon. After allowing the reaction to stir for 10 minutes, adamantane-2-carboxaldehyde (77 mg, 0.467 mmol) in 2 ml ether was added dropwise to effect coupling. The reaction mixture was warmed to room temperature, quenched by partitioning with water, and the collected ether layer was dried through a Na₂SO₄ column and evaporated. The crude alcohol mixture was quickly flashed through a silica gel column, eluting with 10% EtOAc/hexanes to give a purified mixture for spectral analysis. No yield was recorded.

IR(CHCl₃, cm⁻¹): 3593, 3003, 2904, 2893, 1623, 1597, 1511, 1470, 1452, 1433, 1373, 1263, 1170, 1133, 1043, 1023, 846, 828

6-Methoxynaphth-1-yladamant-1'-yl ketone. The mixture of 6-methoxynaphth-1-yladamant-1'-yl alcohols was dissolved in 2 ml acetone and oxidized at 0°C with dropwise addition of Jones reagent. Workup by partitioning the solution between EtOAc/H₂O, evaporating and chromatographing the crude product on silica gel (10% EtOAc/hexanes) afforded pure 6-methoxynaphth-1-yladamant-1'-yl ketone for spectral analysis. No yield was recorded.

IR (CHCl₃, cm⁻¹): 3005, 2905, 2845, 1675 (C=O), 1624, 1595, 1509, 1471, 1455, 1435, 1376, 1260, 1245, 1175, 1096, 1044, 950, 845, 814

NMR (CDCl₃, ppm): 1.50-1.96 (12H); 2.30 (2H, overlapping singlets); 3.30 (1H, s); 3.86 (3H, s); 7.09 (1H, d, J=2.5 Hz); 7.14 (1H, dd, J=9.28, 2.7 Hz); 7.33-7.42 (2H, m); 7.74 (1H, d, J=8.05 Hz); 8.11 (1H, d, J-9.52 Hz)

By proceeding from this point on in the synthesis in the manner described in Example I above, i.e., converting 6-methoxynaphth-1-yladamant-1'-yl ketone to the methyl enol ether, deprotecting with sodium ethanethiolate in dry DMF to give the naphthol followed by in situ acylation with acetic anhydride/pyridine to give methoxy-(6-acetoxynaphth-1'-yl) methylene adamantane, and then photoxygenating this compound's ethylenic double bond, 3-(2'-spiroadamantane)-4-methoxy-4-(6"-acetoxy)naphth-1'-yl-1,2-dioxetane is obtained.

### EXAMPLE IX

### [Synthesis of 3-(2'-spiroadamantane)-4-methoxy-4-(5"-acetoxy)naphth-2'-yl-1,2-dioxetane and 3-(2'-spiroadamantane)-4-methoxy-4-(5"-phosphoryloxy)naphth-2'-yl-1,2-dioxetane].

2-Bromo-5-methoxynaphthalene. Under an argon atmosphere, 60% sodium hydride (1.4g, 0.035 mol) is washed 3 times with hexanes. Sieve dried (3A) DMF (40 ml) is added and the suspension is cooled to 0°C while 6-bromo-1-naphthol (5g., 0.032 mol; Hodgson et al, J.Chem.Soc., 8, 1944) is added with stirring. After hydrogen evolution ceases, dimethyl sulfate (3.4 ml, 0.036 mol) is added dropwise. Stirring is continued in the cold for 8 hours. The reaction mixture is diluted with 250 ml ice cold 0.05N NaOH to yield a gummy precipitate. The aqueous layer is decanted and the residue is triturated several times with dilute NaOH. Plug chromatography on silica gel (15% ethyl acetate-hexanes) affords a good yield of the bromo ether.

R and S 5-methoxynaphth-2-yl-adamanat-2'-yl alcohol. 2-Bromo-5-methoxynaphthalene (500 mg., 2.1 mmol) is dissolved in 25 ml anhydrous ether under argon. The solution is cooled to 0°C and treated with a solution of n-BuLi (1.6M in hexanes, 2 ml) via syringe. The resulting solution is stirred in the cold for 15 minutes prior to the addition of a solution of adamantane-2-carboxaldehyde (416 mg., 2.5 mmol) in 20 ml anhydrous ether. The mixture is slowly warmed to room temperature and quenched with 50 ml 1% NH₄Cl solution. The ether layer is removed, extracted again with water, and dried over Na₂SO₄. The solvent is stripped to afford an oil which is flash chromatographed on silica gel with 10% ethyl acetate-hexanes as eluant. The fractions containing the lower Rf isomeric alcohols are combined and concentrated to yield product in moderate yield.

5-Methoxynaphth-2-yladamant-2'-yl ketone. A solution of the 5-methoxynaphth-2-yl-adamant-2'-yl alcohols (700 mg., 2.17 mmol) in 20 ml acetone is cooled to 0°C and treated dropwise with Jones reagent (23 ml conc. H_{S}O₄ and 26.9g. CrO₃ and water to 100 ml) while stirring vigorously. The rapid reaction is monitored by TLC until the lower Rf starting material is replaced by the less polar ketonic product. The greenish yellow mixture is partitioned between 60 ml ethyl acetate and water. The aqueous layer, which contains some solid, is extracted three times with ethyl acetate (20 ml). The combined organic fractions are washed with saturated NaHCO₃ solution, dried over Na₂SO₄, and stripped to a light yellow oil which exhibits the characteristic carbonyl stretch in the infrared spectrum. The crude product is used directly for conversion to the enol ether.

Methoxy (5-methoxynaphth-2-yl) methylene adamantane. A solution of the crude 5-methoxynaphth-2-yladamant-2'-yl ketone (1g., 3.1 mmol) in 35 ml sieve dried (3A) DMSO at 50°C, is treated with potassium tert-butoxide (0.7g., 6.3 mmol) under a leisurely flow of argon. The colored solution of the enolate anion is stirred for 10 minutes. The solution becomes decolorized during a 15 minute dropwise addition of dimethyl sulfate (595 µl, 6.3 mmol). After cooling to room temperature the solution is stirred overnight and partitioned between ethyl acetate and water. The organic layer is washed three times with water and dried over anhydrous K₂CO₃. Concentration in vacuuo yields an oil which is immediately plug chromatographed on silica gel with 5% ethyl acetate hexanes to yield the enol ether in good yield. TLC analysis in the same solvent system shows that a trace of the more polar ketonic starting material is present as a contaminant.

Methoxy-5(5-hydroxynaphth-2-yl)methylene adamantane. Under an argon atmosphere, 60% sodium hydride (200 mg, 5 mmol) is washed twice with hexanes. Excess solvent is blown off and sieve-dried DMF (15 ml) is added. The resulting slurry is cooled to 0°C with stirring. Ethanethiol (370 µl, 5 mmol) is added dropwise via syringe. After 10 minutes the mixture is warmed to room temperature and stirred until hydrogen evolution abates. Methoxy-(5-methoxynaphth-2-yl)methylene adamantane (750 mg., 2.24 mmol) in 10 ml DMF is added all at once and the resulting solution is refluxed for 3.5 hours. DMF is removed in vacuuo and the residue is partitioned between 5% NH₄Cl and 25 ml ethyl acetate. The aqueous layer is extracted again with another equivalent volume of ethyl acetate. The combined organic layers are washed with 2 X 30 ml water, 1 X 30 ml saturated NaCl, and dried quickly over sodium sulfate. TLC (Whatman K5F, 20% ethyl acetate: CH₂Cl₂) shows the absence of starting material on a single low Rf product. The solvent is stripped and the residue recrystallized from CH₃CN to provide the naphthol in good yield.

3-(2'-Spiroadamantane)-4-methoxy-4-(5"-acetoxy)naphth-2'-yl-1,2-dioxetane. By proceeding from this point on in the synthesis in the manner described in Example I above, i.e., acetylating methoxy-(5-hydroxynaphth-2'-yl)methylene adamantane with acetic anhydride/pyridine to give methoxy-(5-acetoxynaphth2'-yl)methylene adamantane, and then photooxygenating this compound's ethylenic double bond, 3-(2'-spiroadamantane)-4-methoxy-4-(5"-acetoxy)naphth-2'-yl-1,2-dioxetane is obtained.

3-(2'-Spiroadamantane)-4-methoxy-4-(5"-phosphoryloxy)naphth-2'-yl-1,2-dioxetane. Phosphorylation of methoxy-(5-hydroxynaphth-2'-yl)methylene adamantane via the 2-chloro-2-oxo-1,3-dioxa phospholane method described in the aforementioned copending Edwards application to give methoxy-(5-phosphoryloxynaphth-2'-yl)methylene adamantane, and then photooxygenating this compound's ethylenic double bond, gives 3-(2'-spiroadamantane)-4-methoxy-4-(5"-phosphoryloxy)naphth-2'-yl-1,2-dioxetane.

### EXAMPLE X

A dual channel assay for Human Chorionic Gonadotropins (β-chain), β-HCG, and Human Luteinizing Hormones, HLH, is carried out as follows:

### MATERIALS :

A round nylon membrane (approximately 1 inch in diameter) containing two sets of covalently immobilized capture monoclonal antibodies is used, one set for β-HCG available from Medix Biotech, Anti HCG, Cat. No. H298-01, and the second for HLH also available from Medix Biotech, Anti LH, Cat. No. L461-09. This nylon membrane is stored in a foil pouch until used.

Mouse monoclonal anti β-HCG available from Medix Biotech, Cat. No. H298-12, is conjugated with alkaline phosphatase using the glutaraldehyde coupling procedure [Voller, A., et. al., Bull, world Health Org., 53, 55 (1976)] and is used as a detection antibody for β-HCG.

Mouse monoclonal anti HLH available from Medix Biotech, Cat. No. L461-03, is conjugated to carboxylesterase also using the glutaraldehyde coupling procedure referenced above, and is used as a detection antibody for HLH.

The substrate buffer solution used contains 0.05M carbonate, 1mM MgCl₂, 0.1% by weight BSA (pH - 0.5), and 3-(2'-spiroadamantane)-4-methoxy-4(3'-phosphoryloxy)phenyl-1,2-dioxetane disodium salt (50 µg/ml) and 3-(2'-spiroadamantane)-4-methoxy-4-(7"-acetoxy)naphth-2'-yl-1,2-dioxetane (50 µg/ml) as the chemiluminescent substrates.

The wash buffer used contains 0.05M carbonate, 1mM MgCl₂ and 2% by weight BSA (pH = 9.5).

### ASSAY PROCEDURE:

Five drops of a previously collected urine sample are placed onto the center of the assay membrane and allowed to soak into the membrane. Next, five drops of a solution containing β-HCG and HLH conjugated detection antibodies at a concentration of 0.01 millimolar are added to the assay membrane. The liquid is allowed to soak in for at least one minute. Six drops of the wash buffer are slowly added and allowed to soak in and drain for 30 to 60 seconds. Then, five drops of the buffer solution containing chemiluminescent substrates are added and allowed to soak in for approximately one minute.

The assay membrane is placed in a camera luminometer device equipped with pre-exposed calibration scales for β-HCG and LH.

The chemiluminescent light emission, generated as a function of the enzymes, alkaline phosphatase and carboxyl esterase, is imaged through a mask containing two narrow band pass filters (approximately 1cm in diameter). Kodak Wratten Gelatin Filter No. 115 is used to image the green emission from the naphth-2'-yl-1,2-dioxetane substrate, and Kodak Wratten Filter No. 47B is used to isolate the blue emission from the phenyl dioxetane.

The relative levels of β-HCG and HLH present in the sample are determined by a comparison of the appropriate imaged spot brightness with relevant calibration scales.

### EXAMPLE XI

A three-channel analysis for Herpes Simplex Virus, (HSV), Cytomegalovirus, (CMV), and Human Papiloma Virus, (HPV) is carried out as follows:

### MATERIALS:

### "Gene Screen Plus", a positively charged nylon membrane (Dupont NEN Products) is used for hybridization.

The following buffers are used for the assay:

### HSV DNA PROBE ASSAY

### Materials and Buffers:

- Membrane:: Gene Screen Plus membrane.
-
- Buffers:: Denaturation Buffer, 0.5 M NaOH
Neutralization Buffer, 0.4M NaH₂PO₄ (pH = 2.0)
Loading Buffer, 1 part Denaturation Buffer
1 part Neutralization Buffer
Membrane Wetting Buffer 0.4M Tris (pH = 7.5)

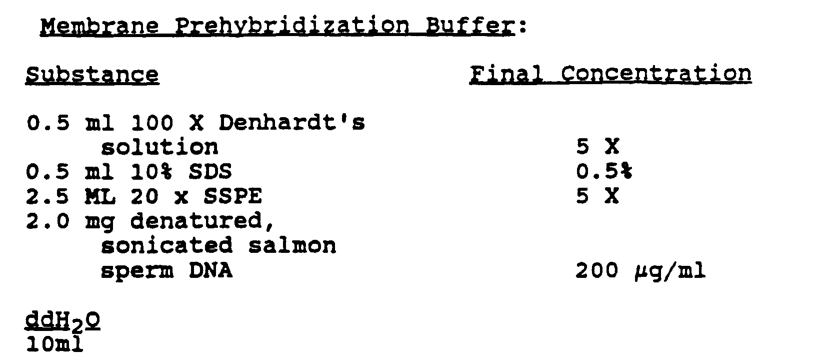

| Membrane Hybridization Buffer : | |
|---|---|
| Substance | Final Concentration |
| 0.5 ml 100 X Denhardt's solution | 5 X |
| 0.5 ml 10% SDS | 0.5% |
| 2.5 ml 20 X SSPE | 5 X |
| 2.0 mg salmon sperm DNA | 200 µg/ml |
| 2.0 ml 50% Dextran sulfate | 10% |
| -- ddH₂O | |
| $\overline{\text{10 ml}}$ | |

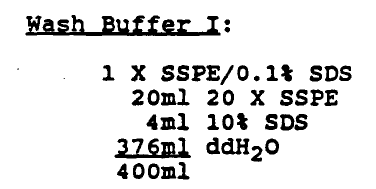

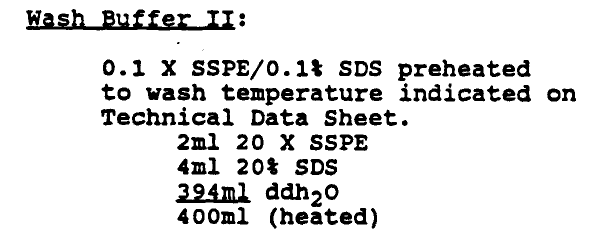

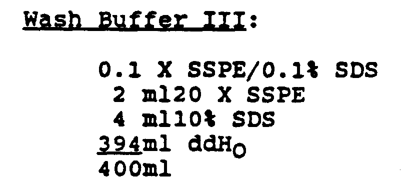

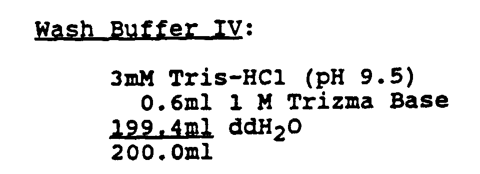

### Was Buffer V:

0.1 M Trizma HCl pH 6.0
100 X Denhart's solution

### Preparation of 100 X Denhart's solution (for 100 mls):

Polyvinylpyrrolidone (2g; mol. wt. 40K; PVP-40) and 2g ficoll are dissolved at a temperature greater than 65°C but less than boiling. The solution is cooled to approximately 40°C, 2g BSA are added and the solution is brought up to the final volume of 100 ml with ddH₂O, aliquoted and stored at -20°C. BSA is easily denatured and in combination with PVP and ficoll it will not go into solution at all if it is not cooled down to -40°C. Hence, the solution is not heated over 40°C when thawing for use.

### Preparation of 20 X SSC solution:

| | |
|---|---|
| 20 X SSC (for 100 ml) | |
| 3.0 M Sodium Chloride | 17.4g |
| 0.3 M Sodium Citrate | 8.8g |

The volume is brought to 100 ml and the solution filtered through a 0.45 µm nitrocellulose filter and stored at room temperature.

### Preparation of 20 X SSPE solution:

20 X SSPE pH 7.4 (for 1 liter)
3.6 M NaCl
200 mM Sodium phosphate 23 g dibasic, 5.92g monobasic
20 mM EDTA 7.44g

These materials are dissolved and adjusted to a pH of 7.4 with 5N NaOH. The volume is then brought to 1 liter and the solution filtered through a 0.45 µm nitrocellulose filter.

### 1 X TE:

- 1 X TE buffer: 10 mM Tris (pH 7.)
1 mM EDTA
Autoclave

The substrate buffer solution used contains 0.05M carbonate, 1 mM MgCl₂, 0.1% by weight BSA (Ph = 9.5), and 3-(2'-spiroadamantane)-4-methoxy-4-(3'-phosphoryloxy)phenyl-1,2-dioxetane disodium salt (50 mg/ml), 3-(2'-spiroadamantane)-4-methoxy-4-(7"-acetoxy)naphth-2'-yl-1,2-dioxetane (50 mg/ml) and 3-(2'-spiroadamantane)-4-metboxy-4-(5"-β-galactosyloxy)naphth-2'-yl-1,2-dioxetane (50 mg/ml) as the chemiluminescent substrates.

### ASSAY PROCEDURE:

Samples (50 µl) containing DNA are denatured by incubation for 10 minutes at room temperature in 200 µl of Denaturation Buffer. Ice cold Neutralization Buffer (250 µl) is then added, and the samples placed on ice.

Nylon membrane is soaked for 15 minutes in Wetting Buffer and then inserted into a vacuum manifold device producing 2 cm diameter spots. Loading Buffer, (200 µl) is then aspirated through each well. The denatured DNA samples are then added to each well and aspirated through the membrane. The manifold is then disassembled and the DNA fixed to the membrane using a UV Transilluminator for 5 minutes. The membrane is then incubated in Prehybridization Buffer at 70°C for 1 hour.

Dots of membrane from the region in which the sample DNA is applied are punched out and inserted into tubes for the remaining steps of the assay. The following enzyme labeled probes are used: probe for HSV labeled with alkaline phosphatase; probe for HPV labeled with β-galactosidase; probe for CMV labeled with carboxylesterase.

The enzyme labeled probes (50 ng of each probe in 200 µl of Hybridization Buffer per tube) are added to each tube and incubated for 2 hours at 70°C. The Hybridization Buffer is then removed and 400 µl of Wash Buffer I added. The tubes are then agitated for 10 minutes at room temperature. Washing is continued by first washing with 400 µl of Wash Buffer II at 50°C for 30 minutes; then with 400 µl of Wash Buffer III at room temperature for 10 minutes; and then with 200 µl of Wash Buffer IV at room temperature.

The membrane is subsequently rinsed with Wash Buffer V at pH 6.0 and placed on a piece of transparent Mylar polyester film. Then, 200 µl of the Substrate Buffer is added and allowed to soak in.

The assay membrane is placed in a camera luminometer device equipped with pre-exposed calibration scales for HSV, HPV and CMV.

The chemiluminescent light emission generated as a function of the enzymes -- alkaline phosphatase, carboxyl esterase and β-galactosidase -- is imaged through a mask containing three narrow bandpass filters (approximately 1cm in diameter). Kodak Wratten Gelatin Filter No. 15 is used to image green emission from the naphthyl acetate dioxetane substrate, Kodak Wratten Filter No. 47B is used to isolate blue emission from the phenyl phosphate dioxetane, and Kodak Wratten Gelatin Filter No. 24 is used to image the orange emission from the naphthyl galactose dioxetane.

The relative levels of HSV, HPV and CMV present in the sample are determined by a comparison of the appropriate image brightness with relevant calibration scales.

The above discussion of this invention is directed primarily to preferred embodiments and practices thereof. It will be readily apparent to those skilled in the art that further changes and modifications in the actual implementation of the concepts described herein can easily be made without departing from the spirit and scope of the invention as defined by the following claims.

## Claims

1. A chemiluminescent 1,2-dioxetane compound having the formula: 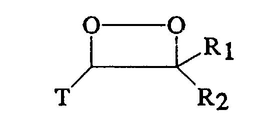 wherein
R₁ is a C₁-C₂₀ alkoxy group;
R₂ is a naphthalene residue bound to the dioxetane ring through a single bond and having a labile ring substituent containing a bond which, when cleaved, renders the naphthalene residue electron-rich to in turn render the dioxetane compound decomposable to emit light, the labile ring substituent's point of attachment to the naphthalene residue, in relation to the naphthalene residue's point of attachment to the dioxetane ring, being such that the total number of ring atoms separating these points of attachment, including the ring atoms at these points of attachment, is an odd whole number; and
T is an adamantyl group.

2. A chemiluminescent 1,2-dioxetane compound according to claim 1 in which the odd whole number is 5 or greater.

3. A chemiluminescent 1,2-dioxetane compound according to claim 1 or 2, in which R₁ is a methoxy group, and T is an adamant-2-ylidene group.

4. A chemiluminescent 1,2-dioxetane compound according to any of claims 1 to 3 in which the labile ring substituent is enzymatically cleavable.

5. A chemiluminescent 1,2-dioxetane compound according to any one of claims 1 to 3 in which the labile ring substituent is a phosphate ester group.

6. A chemiluminescent 1,2-dioxetane compound according to any of claims 1 to 3, in which the labile ring substituent is a galactoside group.

7. A chemiluminescent 1,2-dioxetane compound according to any of claims 1 to 3, in which the labile ring substituent is chemically cleavable.

8. A 3-(2'-spiroadamantane)-4-methoxy-4-(6"-phosphoryloxy)naphth-1'-yl-1,2-dioxetane salt.

9. A 3-(2'-spiroadamantane)-4-methoxy-4-(7"-phosphoryloxy)naphth-2'-yl-1,2-dioxetane salt.

10. A 3-(2'-spiroadamantane)-4-methoxy-4-(8"-phosphoryloxy)naphth-1'-yl-1,2-dioxetane salt.

11. A 3-(2'-spiroadamantane)-4-methoxy-4-(5"-phosphoryloxy)naphth-2'-yl-1,2-dioxetane salt.

12. The disodium salt of a 1,2-dioxetane compound as claimed in claim 7.

13. A method for generating light which comprises:
(a) providing a chemiluminescent 1,2-dioxetane compound having the formula: 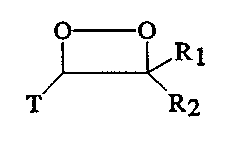 wherein
R₁ is a C₁-C₂₀ alkoxy group;
R₂ is a naphthalene residue having a labile ring substituent containing a bond which, when cleaved, renders the naphthalene residue electron-rich to in turn render the dioxetane compound decomposable to emit light, the labile ring substituent's point of attachment to the naphthalene residue, in relation to the naphthalene residue's point of attachment to the dioxetane ring, being such that the total number of ring atoms separating these points of attachment, including the ring atoms at these points of attachment, is an odd whole number; and
T is an adamantyl group; and
(b) decomposing the 1,2-dioxetane compound by cleaving the labile ring substituent's bond.

14. A method for generating light according to claim 13 in which the labile ring substituent's bond is cleaved by an enzyme.

15. A method for generating light according to claim 14 in which the 1,2-dioxetane compound is a 3-(2'-spiroadamantane)-4-methoxy-4-(6"-phosphoryloxy)naphth-1'-yl-1,2-dioxetane salt, a 3-(2'-spiroadamantane)-4-methoxy-4- (7"-phosphoryloxy)naphth-2'-yl-1,2-dioxetane salt, a 3-(2'-spiroadamantane)-4-methoxy-4-(8"-phosphoryloxy)naphth-1'-yl-1,2-dioxetane salt or a 3-(2'-spiroadamantane)-4-methoxy-4-(5"-phosphoryloxy)naphth-2'-yl-1,2-dioxetane salt.

16. A method for generating light according to claim 15 in which the 1,2-dioxetane salt is a disodium salt.

17. A process for determining the presence, concentration or structure of an analyte, which process comprises detecting light released by the decomposition of a chemiluminescent compound, characterised in that there is used as chemiluminescent compound at least one chemiluminescent 1,2-dioxetane compound as claimed in any one of claims 1 to 12.

18. A process according to claim 17 which process is carried out as a step in an immunoassay.

19. A process according to claim 18 in which the immunoassay is for the detection of a specific binding pair comprising an antigen and an antibody.

20. A process according to claim 18 in which an enzyme is used as label in the assay.

21. A process according to claim 18 in which the chemiluminescent 1,2-dioxetane compound is used as label in the assay.

22. A process according to claim 18 in which the chemiluminescent 1,2-dioxetane compound is enzymatically cleavable.

23. A process according to claim 18 in which the chemiluminescent 1,2-dioxetane compound is chemically cleavable.

24. A process according to claim 18 in which the immunoassay is for the detection of an enzyme.

25. A process according to claim 18 in which the immunoassay is for the detection of a hormone.

26. A process according to claim 17 which process is carried out as a step in a chemical assay.

27. A process according to claim 26 in which the chemical assay is for the detection of a chemical substance which, during the assay, is caused to decompose to form a substance capable of causing the chemiluminescent 1,2-dioxetane compound to decompose.

28. A process according to claim 27 in which the chemical substance is glucose.

29. A process according to claim 27 in which the chemical substance is cholesterol.

30. A process according to claim 17 in which the process carried out is a nucleic acid probe assay.

31. A process according to claim 30 in which the nucleic acid probe assay is for the detection of a virus.

32. A process according to claim 17 in which the process carried out is a histocompatibility assay.

33. A process according to claim 17 in which the process carried out is a technique for studying the microstructure of a macromolecule.

34. A process according to claim 17 in which the process carried out is a multi-channel assay carried out in the presence of at least two of the chemiluminescent 1,2-dioxetane compounds as claimed in claim 1 as substrates, each of which upon decomposition emits light of a different wavelength from the other(s) and each of which has a labile ring substituent cleavable by a different means from the other(s).

35. A process according to claim 17 in which the process carried out is a multi-channel assay carried out in the presence of at least one of the chemiluminescent 1,2-dioxetane compounds as claimed in claim 1 and at least one other chemiluminescent compound as substrates, each of which chemiluminescent 1,2-dioxetane compounds and other chemiluminescent compounds emit light of a different wavelength from the other(s) and each of which is cleavable by different means from the other(s) to produce a light-emitting fluorophore moiety.

36. A process according to claim 35 in which the multi-channel assay is an immunoassay for the quantitation of human chorionic gonadotropin and human luteinizing hormone contained in a single sample and is carried out in the presence of 3-(2'-spiroadamantane)-4-methoxy-4-(3'-phosphoryloxy)phenyl-1,2-dioxetane disodium salt and 3-(2'-spiroadamantane)-4-methoxy-4-(7"-acetoxy)naphth-2'-yl-1,2-dioxetane as the chemiluminescent substrates.

37. A process according to claim 36 in which the phosphoryloxyphenyl dioxetane chemiluminescent substrate is cleaved by alkaline phosphatase conjugated to mouse monoclonal anti-β-HCG and the acetoxynaphthyl dioxetane chemiluminescent substrate is cleaved by carboxylesterase conjugated to mouse monoclonal anti-HLH.

38. A process according to claim 35 in which the multi-channel assay is a nucleic acid probe assay for the quantitation of herpes simplex virus, cytomegalovirus and human papiloma virus contained in a single sample and is carried out in the presence of 3-(2'-spiroadamantane)-4-methoxy-4-(3'-phosphoryloxy)phenyl-1,2-dioxetane disodium salt, 3-(2'-spiroadamantane)-4-methoxy-4-(7"-acetoxy)naphth-2'-yl-1,2-dioxetane and 3-(2'-spiroadamantane)-4-methoxy-4-(5"-β-galactosyloxy)naphth-2'-yl-1,2-dioxetane as the chemiluminescent substrates.

39. A process according to claim 38 in which the phosphoryloxyphenyl dioxetane chemiluminescent substrate is cleaved by a DNA probe for HSV labelled with alkaline phosphatase, the acetoxynaphthyl dioxetane chemiluminescent substrate is cleaved by a DNA probe for CMV labelled with carboxylesterase and the β-galactosyloxynaphthyl dioxetane chemiluminescent substrate is cleaved by a DNA probe for HPV labelled with β-galactosidase.

40. A compound having the general formula: 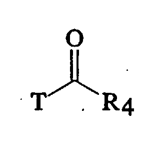 wherein T is an adamantyl group and R₄ is a naphthalene having an ether substituent, the total number of carbon atoms separating the ring carbon atom to which said ether substituent is attached and the ring carbon atom through which R₄ is attached to the remainder of the compound, including the carbon atoms at said points of attachment, being an odd whole number.

## Patentansprüche

1. Chemilumineszierende 1,2-Dioxetan-Verbindung mit der Formel: 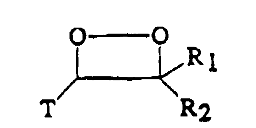 worin
R₁ eine C₁-C₂₀-Alkoxygruppe ist;
R₂ ein Naphthalen-Rest ist, der an den Dioxetan-Ring durch eine Einfachbindung gebunden ist und einen labilen Ring-Substituent aufweist, der eine Bindung enthält, welche, wenn gespalten, den Naphthalen-Rest elektronenreich macht, damit dies wiederum die Dioxetan-Verbindung zerlegbar macht, um Licht zu emittieren, wobei die Verknüpfungsstelle des labilen Ring-Substituent an den Naphthalen-Rest, bezogen auf die Verknüpfungsstelle des Naphthalen-Rests an den Dioxetan-Ring, derart ist, daß die Gesamtzahl der Ringatome, die diese Verknüpfungsstellen trennen, einschließlich der Ringatome an diesen Verknüpfungsstellen, eine ungerade ganze Zahl ist; und
T eine Adamantylgruppe ist.

2. Chemilumineszierende 1,2-Dioxetan-Verbindung nach Anspruch 1, bei welcher die ungerade ganze Zahl 5 oder größer ist.

3. Chemilumineszierende 1,2-Dioxetan-Verbindung nach Anspruch 1 oder 2, bei welcher R₁ eine Methoxygruppe ist und T eine Adamant-2-ylidengruppe ist.

4. Chemilumineszierende 1,2-Dioxetan-Verbindung nach einem der Ansprüche 1 bis 3, bei welcher der labile Ring-Substituent enzymatisch spaltbar ist.

5. Chemilumineszierende 1,2-Dioxetan-Verbindung nach einem der Ansprüche 1 bis 3, bei welcher der labile Ring-Substituent eine Phosphatestergruppe ist.

6. Chemilumineszierende 1,2-Dioxetan-Verbindung nach einem der Ansprüche 1 bis 3, bei welcher der labile Ring-Substituent eine Galactosidgruppe ist.

7. Chemilumineszierende 1,2-Dioxetan-Verbindung nach einem der Ansprüche 1 bis 3, bei welcher der labile Ring-Substituent chemisch spaltbar ist.

8. 3-(2'-Spiroadamantan)-4-methoxy-4-(6"-phosphoryloxy)-naphth-1'-yl-1,2-dioxetansalz.

9. 3-(2'-Spiroadamantan)-4-methoxy-4-(7"-phosphoryloxy)-naphth-2'-yl-1,2-dioxetansalz.

10. 3-(2'-Spiroadamantan)-4-methoxy-4-(8"-phosphoryloxy)-naphth-1'-yl-1,2-dioxetansalz.

11. 3-(2'-Spiroadamantan)-4-methoxy-4-(5"-phosphoryloxy)-naphth-2'-yl-1,2-dioxetansalz.

12. Das Dinatriumsalz einer 1,2-Dioxetan-Verbindung nach Anspruch 7.

13. Verfahren zur Erzeugung von Licht, welches umfaßt:
(a) Bereitstellen einer chemilumineszierenden 1,2-Dioxetan-Verbindung mit der Formel: 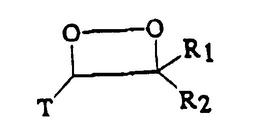 worin
R₁ eine C₁-C₂₀-Alkoxygruppe ist;
R₂ ein Naphthalen-Rest ist mit einem labilen Ring-Substituent, der eine Bindung enthält, welche, wenn gespalten, den Naphthalen-Rest elektronenreich macht, damit dies wiederum die Dioxetan-Verbindung zerlegbar macht, um Licht zu emittieren, wobei die Verknüpfungsstelle des labilen Ring-Substituent an den Naphthalen-Rest, bezogen auf die Verknüpfungsstelle des Naphthalen-Rests an den Dioxetan-Ring, derart ist, daß die Gesamtzahl der Ringatome, die diese Verknüpfungsstellen trennen, einschließlich der Ringatome an diesen Verknüpfungsstellen, eine ungerade ganze Zahl ist; und
T eine Adamantylgruppe ist; und
(b) Zerlegen der 1,2-Dioxetan-Verbindung durch Spalten der Bindung des labilen Ring-Substituent.

14. Verfahren zur Erzeugung von Licht nach Anspruch 13, bei welchem die Bindung des labilen Ring-Substituent durch ein Enzym gespalten wird.

15. Verfahren zur Erzeugung von Licht nach Anspruch 14, bei welchem die 1,2-Dioxetan-Verbindung ein 3-(2'-Spiroadamantan)-4-methoxy-4-(6"-phosphoryloxy)naphth-1'-yl-1,2-dioxetansalz, ein 3-(2'-Spiroadamantan)-4-methoxy-4-(7"-phosphoryloxy)naphth-2'-yl-1,2-dioxetansalz, ein 3-(2'-Spiroadamantan)-4-methoxy-4-(8"-phosphoryloxy)naphth-1'-yl-1,2-diaxetansalz oder ein 3-(2'-Spiroadamantan)-4-methoxy-4-(5"-phosphoryloxy)naphth-2'-yl-1,2-dioxetansalz ist.

16. Verfahren zur Erzeugung von Licht nach Anspruch 15, bei welchem das 1,2-Dioxetansalz ein Dinatriumsalz ist.

17. Verfahren zur Bestimmung von Vorhandensein, Konzentration oder Struktur eines Analyts, welches Verfahren das Nachweisen von Licht umfaßt, das durch die Zerlegung einer chemilumineszierenden Verbindung freigesetzt wird, dadurch gekennzeichnet, daß als chemilumineszierende Verbindung mindestens eine chemilumineszierende 1,2-Dioxetan-Verbindung nach einem der Ansprüche 1 bis 12 verwendet wird.

18. Verfahren nach Anspruch 17, welches Verfahren als ein Schritt in einem Immunoassay ausgeführt wird.

19. Verfahren nach Anspruch 18, bei welchem der Immunoassay zum Nachweisen eines spezifischen Bindungspaars dient, umfassend ein Antigen und einen Antikörper.

20. Verfahren nach Anspruch 18, bei welchem ein Enzym als Markierung bei dem Assay verwendet wird.

21. Verfahren nach Anspruch 18, bei welchem die chemilumineszierende 1,2-Dioxetan-Verbindung als Markierung bei dem Assay verwendet wird.

22. Verfahren nach Anspruch 18, bei welchem die chemilumineszierende 1,2-Dioxetan-Verbindung enzymatisch spaltbar ist.

23. Verfahren nach Anspruch 18, bei welchem die chemilumineszierende 1,2-Dioxetan-Verbindung chemisch spaltbar ist.

24. Verfahren nach Anspruch 18, bei welchem der Immunoassay zum Nachweisen eines Enzyms dient.

25. Verfahren nach Anspruch 18, bei welchem der Immunoassay zum Nachweisen eines Hormons dient.

26. Verfahren nach Anspruch 17, welches Verfahren als eine Stufe bei einem chemischen Assay durchgeführt wird.

27. Verfahren nach Anspruch 26, bei welchem der chemische Assay zum Nachweisen einer chemischen Substanz dient, welche während des Assays zur Zerlegung gebracht wird, um eine Substanz zu bilden, die die Zerlegung der chemilumineszierenden 1,2-Dioxetan-Verbindung bewirken kann .

28. Verfahren nach Anspruch 27, bei welchem die chemische Substanz Glucose ist.

29. Verfahren nach Anspruch 27, bei welchem die chemische Substanz Cholesterol ist.

30. Verfahren nach Anspruch 17, bei welchem das durchgeführte Verfahren ein Nukleinsäuresonden-Assay ist.

31. Verfahren nach Anspruch 30, bei welchem der Nukleinsäuresonden-Assay zum Nachweisen eines Virus dient.

32. Verfahren nach Anspruch 17, bei welchem das durchgeführte Verfahren ein Histokompatibilitäts-Assay ist.

33. Verfahren nach Anspruch 17, bei welchem das durchgeführte Verfahren eine Methode zum Untersuchen der Mikrostruktur eines Makromoleküls ist.

34. Verfahren nach Anspruch 17, bei welchem das durchgeführte Verfahren ein Mehrkanal-Assay ist, durchgeführt in Gegenwart von mindestens zwei der chemilumineszierenden 1,2-Dioxetan-Verbindungen nach Anspruch 1 als Substrate, wobei jede davon bei Zerlegung Licht einer von der/den anderen verschiedenen Wellenlänge emittiert und jede davon einen labilen Ring-Substituent aufweist, der mittels einer von der/den anderen verschiedenen Methode spaltbar ist.

35. Verfahren nach Anspruch 17, bei welchem das durchgeführte Verfahren ein Mehrkanal-Assay ist, durchgeführt in Gegenwart von mindestens einer der chemilumineszierenden 1,2-Dioxetan-Verbindungen nach Anspruch 1 und mindestens einer anderen chemilumineszierenden Verbindung als Substrate, wobei jede der chemilumineszierenden 1,2-Dioxetan-Verbindungen und anderen chemilumineszierenden Verbindungen Licht einer von der/den anderen verschiedenen Wellenlänge emittiert und jede davon mittels einer von der/den anderen verschiedenen Methode spaltbar ist, um eine Licht-emittierende Fluorophor-Komponente zu erzeugen.

36. Verfahren nach Anspruch 35, bei welchem der Mehrkanal-Assay ein Immunoassay zum Quantifizieren von in einer Einzelprobe enthaltenem chorialem Human-Gonadotropin und humanem luteinisierendem Hormon ist und durchgeführt wird in Gegenwart von 3-(2'-Spiroadamantan)-4-methoxy-4-(3'-phosphoryloxy)phenyl-1,2-dioxetan-Dinatriumsalz und 3-(2'-Spiroadamantan)-4-methoxy-4-(7"-acetoxy)naphth-2'-yl-1,2-dioxetan als den chemilumineszierenden Substraten.

37. Verfahren nach Anspruch 36, bei welchem das chemilumineszierende Phosphoryloxyphenyldioxetan-Substrat durch alkalische Phosphatase gespalten wird, die an monoklonales Maus-Anti-β-HCG konjugiert ist, und das chemilumineszierende Acetoxynaphthyldioxetan-Substrat durch Carboxylesterase gespalten wird, das an monoklonales Maus-Anti-HLH konjugiert ist.

38. Verfahren nach Anspruch 35, bei welchem der Mehrkanal-Assay ein Nukleinsäuresonden-Assay zum Quantifizieren von in einer Einzelprobe enthaltenem Herpes simplex Virus, Cytomegalievirus und humanes Papillomavirus ist und durchgeführt wird in Gegenwart von 3-(2'-Spiroadamantan)-4-methoxy-4-(3'-phosphoryloxy)phenyl-1,2-dioxetan-Dinatriumsalz, 3-(2'-Spiroadamantan)-4-methoxy-4-(7"-acetoxy)naphth-2'-yl-1,2-dioxetan und 3-(2'-Spiroadamantan)-4-methoxy-4-(5"-β-galactosyloxy)naphth-2'-yl-1,2-dioxetan als den chemilumineszierenden Substraten.

39. Verfahren nach Anspruch 38, bei welchem das chemilumineszierende Phosphoryloxyphenyldioxetan-Substrat durch eine DNA-Sonde für HSV, markiert mit alkalischer Phosphatase, gespalten wird, das chemilumineszierende Acetoxynaphthyldioxetan-Substrat durch eine DNA-Sonde für CMV, markiert mit Carboxylesterase, gespalten wird, und das chemilumineszierende β-Galactasyloxynaphthyldioxetan-Substrat durch eine DNA-Sonde für HPV, markiert mit β-Galactosidase, gespalten wird.

40. Verbindung der allgemeinen Formel: 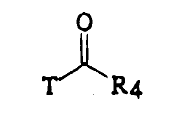 worin T eine Admantylgruppe und R₄ ein Naphthalen mit einem Ether-Substituent ist, wobei die Gesamtzahl der Kohlenstoffatome, die das Ring-Kohlenstoffatom, mit dem der Ether-Substituent verknüpft ist, und das Ring-Kohlenstoffatom, durch das R₄ mit dem Rest der Verbindung verknüpft ist, trennen, einschließlich der Kohlenstoffatome an diesen Verknüpfungsstellen, eine ungerade ganze Zahl ist.

## Revendications

1. Composé 1,2-dioxétane chimioluminescent, correspondant à la formule : 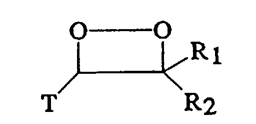 dans laquelle
R₁ est un groupe alcoxy en C₁-C₂₀ ;
R₂ est un résidu naphtalène qui est lié au cycle dioxétane par une liaison simple et qui possède un substituant labile ayant une liaison qui, lorsqu'elle est coupée, enrichit en électrons le résidu naphtalène qui, à son tour, rend le composé dioxétane décomposable de façon à ce qu'il émette de la lumière, le point de fixation du substituant labile au résidu naphtalène, par rapport au point de fixation du résidu naphtalène au cycle dioxétane, étant tel que le nombre total d'atomes séparant ces points de fixation, y compris les atomes à ces points de fixation, est un nombre entier impair ; et
T est un groupe adamantyle.

2. Composé 1,2-dioxétane chimioluminescent selon la revendication 1, dans lequel le nombre entier impair est supérieur ou égal à 5.

3. Composé 1,2-dioxétane chimioluminescent selon la revendication 1 ou 2, dans lequel R₁ est un groupe méthoxy et T est un groupe adamant-2-ylidène.

4. Composé 1,2-dioxétane chimioluminescent selon l'une quelconque des revendications 1 à 3, dans lequel le substituant labile peut être coupé par voie enzymatique.

5. Composé 1,2-dioxétane chimioluminescent selon l'une quelconque des revendications 1 à 3, dans lequel le substituant labile est un groupe phosphate ester.

6. Composé 1,2-dioxétane chimioluminescent selon l'une quelconque des revendications 1 à 3, dans lequel le substituant labile est un groupe galactoside.

7. Composé 1,2-dioxétane chimioluminescent selon l'une quelconque des revendications 1 à 3, dans lequel le substituant labile peut être coupé par voie chimique.

8. Sel de 3-(2'-spiroadamantane)-4-méthoxy-4-(6"-phosphoryloxy)napht-1'-yl-1,2-dioxétane.

9. Sel de 3-(2'-spiroadamantane)-4-méthoxy-4-(7"-phosphoryloxy)napht-2'-yl-1,2-dioxétane.

10. Sel de 3-(2'-spiroadamantane)-4-méthoxy-4-(8"-phosphoryloxy)napht-1'-yl-1,2-dioxétane.

11. Sel de 3-(2'-spiroadamantane)-4-méthoxy-4-(5"-phosphoryloxy)napht-2'-yl-1,2-dioxétane.

12. Sel disodique d'un composé 1,2-dioxétane tel que revendiqué dans la revendication 7.

13. Procédé pour produire de la lumière, qui comprend les étapes consistant à :
a) fournir un composé 1,2-dioxétane chimioluminescent de formule : 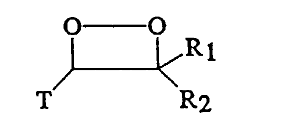 dans laquelle
R₁ est un groupe alcoxy en C₁-C₂₀ ;
R₂ est un résidu naphtalène possédant un substituant labile ayant une liaison qui, lorsqu'elle est coupée, enrichit en électrons le résidu naphtalène qui, à son tour, rend le composé dioxétane décomposable de façon à ce qu'il émette de la lumière, le point de fixation du substituant labile au résidu naphtalène, par rapport au point de fixation du résidu naphtalène au cycle dioxétane, étant tel que le nombre total d'atomes séparant ces points de fixation, y compris les atomes à ces points de fixation, est un nombre entier impair ; et
T est un groupe adamantyle ; et
b) décomposer le composé 1,2-dioxétane en coupant la liaison du substituant labile.

14. Procédé pour produire de la lumière selon la revendication 13, dans lequel la liaison du substituant labile est coupée par une enzyme.

15. Procédé pour produire de la lumière selon la revendication 14, dans lequel le composé 1,2-dioxétane est un sel de 3-(2'-spiroadamantane)-4-méthoxy-4-(6"-phosphoryloxy)napht-1'-yl-1,2-dioxétane, un sel de 3-(2'-spiroadamantane)-4-méthoxy-4-(7"-phosphoryloxy)napht-2'-yl-1,2-dioxétane, un sel de 3-(2'-spiroadamantane)-4-méthoxy-4-(8"-phosphoryloxy)napht-1'-yl-1,2-dioxétane ou un sel de 3-(2'-spiroadamantane)-4-méthoxy-4-(5"-phosphoryloxy)napht-2'-yl-1,2-dioxétane.

16. Procédé pour produire de la lumière selon la revendication 15, dans lequel le sel de 1,2-dioxétane est un sel disodique.

17. Procédé pour déterminer la présence, la concentration ou la structure d'un analyte, qui comprend la détection de la lumière émise par la décomposition d'un composé chimioluminescent, caractérisé en ce que, en tant que composé chimioluminescent, on utilise au moins un composé 1,2-dioxétane chimioluminescent tel que revendiqué dans l'une quelconque des revendications 1 à 12.

18. Procédé selon la revendication 17, qui constitue une étape dans un test immunologique.

19. Procédé selon la revendication 18, dans lequel le test immunologique est utilisé pour détecter un couple de liaison spécifique comprenant un antigène et un anticorps.

20. Procédé selon la revendication 18, dans lequel une enzyme est utilisée en tant que marqueur dans le test.

21. Procédé selon la revendication 18, dans lequel le composé 1,2-dioxétane chimioluminescent est utilisé en tant que marqueur dans le test.

22. Procédé selon la revendication 18, dans lequel le composé 1,2-dioxétane chimioluminescent peut être coupé par voie enzymatique.

23. Procédé selon la revendication 18, dans lequel le composé 1,2-dioxétane chimioluminescent peut être coupé par voie chimique.

24. Procédé selon la revendication 18, dans lequel le test immunologique est utilisé pour la détection d'une enzyme.

25. Procédé selon la revendication 18, dans lequel le test immunologique est utilisé pour la détection d'une hormone.

26. Procédé selon la revendication 17, qui constitue une étape dans un test chimique.

27. Procédé selon la revendication 26, dans lequel le test chimique est utilisé pour la détection d'une substance chimique dont on provoque la décomposition, pendant le test, de façon à ce qu'elle forme une substance capable de provoquer la décomposition du composé 1,2-dioxétane chimioluminescent.

28. Procédé selon la revendication 27, dans lequel la substance chimique est du glucose.

29. Procédé selon la revendication 27, dans lequel la substance chimique est du cholestérol.

30. Procédé selon la revendication 17, dans lequel le procédé mis en oeuvre est un test effectué à l'aide d'une sonde d'acide nucléique.

31. Procédé selon la revendication 30, dans lequel le test effectué à l'aide d'une sonde d'acide nucléique est utilisé pour la détection d'un virus.

32. Procédé selon la revendication 17, dans lequel le procédé mis en oeuvre est un test d'histocompatibilité.

33. Procédé selon la revendication 17, dans lequel le procédé mis en oeuvre est une technique pour étudier la microstructure d'une macromolécule.

34. Procédé selon la revendication 17, dans lequel le procédé mis en oeuvre est un test à voies multiples effectué en présence d'au moins deux des composés 1,2-dioxétane chimioluminescents tels que revendiqués dans la revendication 1, en tant que substrats, chacun d'entre eux émettant, lors de sa décomposition, de la lumière ayant une longueur d'onde différente de celle émise par l'autre (les autres) et chacun d'entre eux possédant un substituant labile pouvant être coupé par un moyen différent de celui permettant de couper le substituant labile de l'autre (des autres).

35. Procédé selon la revendication 17, dans lequel le procédé mis en oeuvre est un test à voies multiples effectué en présence d'au moins un des composés 1,2-dioxétane chimioluminescents tels que revendiqués dans la revendication 1 et d'au moins un autre composé chimioluminescent, en tant que substrats, chacun des composés 1,2-dioxétane chimioluminescents et autres composés chimioluminescents émettant de la lumière ayant une longueur d'onde différente de celle émise par l'autre (les autres) et chacun d'entre eux pouvant être coupé par un moyen différent de celui permettant de couper l'autre (les autres) de façon à produire une partie fluorogène émettant de la lumière.

36. Procédé selon la revendication 35, dans lequel le test à voies multiples est un test immunologique utilisé pour la détermination quantitative de la gonadotrophine chorionique humaine et de l'hormone lutéinisante humaine contenues dans un même échantillon et il est réalisé en présence de sel disodique du 3-(2'-spiroadamantane)-4-méthoxy-4-(3'-phosphoryloxy)phényl-1,2-dioxétane et de 3-(2'-spiroadamantane)-4-méthoxy-4-(7"-acétoxy)napht-2'-yl-1,2-dioxétane en tant que substrats chimioluminescents.

37. Procédé selon la revendication 36, dans lequel le substrat chimioluminescent phosphoryloxyphényldioxétane est coupé par une phosphatase alcaline conjuguée à un anti-β-HCG monoclonal murin et le substrat chimioluminescent acétoxynaphtyldioxétane est coupé par une carboxylestérase conjuguée à un anti-HLH monoclonal murin.

38. Procédé selon la revendication 35, dans lequel le test à voies multiples est un test effectué à l'aide d'une sonde d'acide nucléique pour quantifier le virus de l'herpes simplex, le cytomégalovirus et le virus du papillome humain contenus dans un même échantillon et il est réalisé en présence de sel disodique du 3-(2'-spiroadamantane)-4-méthoxy-4-(3'-phosphoryloxy)phényl-1,2-dioxétane, de 3-(2'-spiroadamantane)-4-méthoxy-4-(7"-acétoxy)napht-2'-yl-1,2-dioxétane et de 3-(2'-spiroadamantane)-4-méthoxy-4-(5"-β-galactosyloxy)napht-2'-yl-1,2-dioxétane en tant que substrats chimioluminescents.

39. Procédé selon la revendication 38, dans lequel le substrat chimioluminescent phosphoryloxyphényldioxétane est coupé par une sonde d'ADN spécifique du HSV marquée avec une phosphatase alcaline, le substrat chimioluminescent acétoxynaphtyldioxétane est coupé par une sonde d'ADN spécifique du CMV marquée avec une carboxylestérase et le substrat chimioluminescent β-galactosyloxynaphtyldioxétane est coupé par une sonde d'ADN spécifique du HPV marquée avec une β-galactosidase.

40. Composé de formule générale : 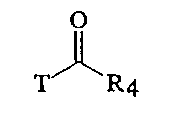 dans laquelle T est un groupe adamantyle et R₄ est un groupe naphtalène possédant un substituant éther, le nombre total d'atomes de carbone séparant l'atome de carbone auquel ledit substituant éther est fixé et l'atome de carbone par l'intermédiaire duquel R₄ est fixé au reste du composé, y compris les atomes de carbone auxdits points de fixation, étant un nombre entier impair.
